# EUROPEAN PATENT SPECIFICATION

(11) **EP 4 400 501 B1**
(45) Date of publication and mention of the grant of the patent: **08.04.2026**
(21) Application number: 23863463.8
(22) Date of filing: 05.09.2023
(51) Int. Cl.: C07D 251/24, C07D 403/10, H10K 85/60, H10K 50/16, H10K 50/17

(54) **NOVEL COMPOUND AND ORGANIC LIGHT EMITTING DEVICE COMPRISING SAME**
NEUARTIGE VERBINDUNG UND ORGANISCHE LICHTEMITTIERENDE VORRICHTUNG DAMIT
NOUVEAU COMPOSÉ ET DISPOSITIF ÉLECTROLUMINESCENT ORGANIQUE LE COMPRENANT

(30) Priority: 05.09.2022 KR 20220112123
(43) Date of publication of application: 17.07.2024
(73) Proprietor: LG Chem, Ltd., Yeongdeungpo-gu Seoul 07336 (KR)
(72) Inventor: HEO, Dong Uk, Daejeon 34122 (KR); HAN, Su Jin, Daejeon 34122 (KR); HONG, Sung Kil, Daejeon 34122 (KR); LEE, Jae Tak, Daejeon 34122 (KR); YOON, Jung Min, Daejeon 34122 (KR); YUN, Heekyung, Daejeon 34122 (KR)
(74) Representative: Hoffmann Eitle
(86) International application number: PCT/KR2023/013239
(87) International publication number: WO 2024/053991

(56) References cited:
- WO-A1-2020/071627
- CN-A- 112 047 930
- CN-A- 114 057 660
- JP-A- 2021 070 682
- KR-A- 20140 087 804
- KR-A- 20190 143 282
- KR-A- 20220 065 123
- KR-B1- 102 566 290

## Description

### [TECHNICAL FIELD]

### CROSS-REFERENCE TO RELATED APPLICATION(S)

This application claims priority benefit of Korean Patent Application No. 10-2022-0112123 filed on September 5, 2022 in the Korean Intellectual Property Office.

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuous need to develop a new material for the organic material used in the organic light emitting device as described above.

### [Prior Art Literature]

### [Patent Literature]

(Patent Literature 0001) Korean Unexamined Patent Publication No. 10-2000-0051826.

WO2020/071627 discloses naphthalene derivatives useful for OLEDs.

### [DETAILED DESCRIPTION OF THE INVENTION]

### [Technical Problem]

It is an object of the present disclosure to provide a novel compound and an organic light emitting device including the same.

### [Technical Solution]

Provided herein is a compound represented by the following Chemical Formula 1: 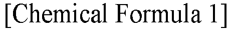 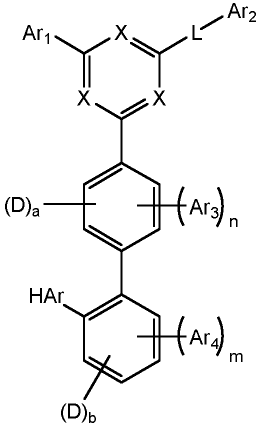 wherein, in Chemical Formula 1,
each X is independently N, CH, or CD, with the proviso that two or more of the X are N,
L is a single bond, or a substituted or unsubstituted C₆₋₆₀ arylene,
Ar₁ and Ar₂ are each independently a substituted or unsubstituted C₆₋₆₀ aryl,
Ar₃ and Ar₄ are each independently phenyl unsubstituted or substituted with at least one deuterium,
any one of Ar₁ to Ar₄ is substituted with cyano,
HAr is pyrimidinyl which is unsubstituted or substituted with 1 to 3 substituent groups each independently selected from the group consisting of deuterium, a substituted or unsubstituted C₁₋₆₀ alkyl, and a substituted or unsubstituted C₆₋₆₀ aryl; triazinyl which is substituted with two substituted or unsubstituted C₆₋₆₀ aryls; or quinazolinyl which is substituted with one substituted or unsubstituted C₆₋₆₀ aryl,
a and b are each independently an integer from 0 to 4,
n and m are each independently 0 or 1,
with the proviso that n+m is 1,
a+n is an integer from 0 to 4, and
b+m is an integer from 0 to 4.

Also provided herein is an organic light emitting device comprising: a first electrode; a second electrode that is provided opposite to the first electrode; and one or more organic material layers that are provided between the first electrode and the second electrode, wherein at least one layer of the organic material layers comprises the compound represented by Chemical Formula 1.

### [ADVANTAGEOUS EFFECTS]

The compound represented by Chemical Formula 1 as mentioned above can be used as a material of an organic material layer in an organic light emitting device, and can improve the efficiency, achieve low driving voltage and/or improve lifetime characteristics in the organic light emitting device. In particular, the compound represented by Chemical Formula 1 can be used as hole injection, hole transport, hole injection and transport, light emission, electron transport, or electron injection materials.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 3, an electron transport and injection layer 7, and a cathode 4.

### [DETAILED DESCRIPTION OF THE EMBODIMENTS]

Hereinafter, embodiments of the present disclosure will be described for a more complete understanding of the disclosed subject matter.

As used herein, the notation 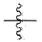 mean a bond connected to another substituent group, and "D" means deuterium.

As used herein, the term "substituted or unsubstituted" means being unsubstituted or substituted with one or more substituent groups selected from the group consisting of deuterium; a halogen group; a cyano group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heterocyclic group containing one or more of N, O and S atoms, or being unsubstituted or substituted with a substituent to which two or more substituents of the above-exemplified substituents are linked. For example, "a substituent in which two or more substituents are linked" may be a biphenyl group. Namely, a biphenylyl group may be an aryl group, or it may also be interpreted as a substituent group in which two phenyl groups are linked. In one example, the term "substituted or unsubstituted" may be understood to mean "being unsubstituted or substituted with one or more substituent groups selected from the group consisting of deuterium, halogen, cyano, a C₁₋₁₀ alkyl, a C₁₋₁₀ alkoxy and a C₆₋₂₀ aryl", or "being unsubstituted or substituted with one or more substituent groups selected from the group consisting of deuterium, halogen, cyano, methyl, ethyl, phenyl and naphthyl". Further, the term "substituted with one or more substituent groups" as used herein may be understood to mean "being substituted with mono to the maximum number of available substitutable hydrogen atoms". Alternatively, the term "substituted with one or more substituent groups" as used herein may be understood to mean "being substituted with 1 to 5 substituent groups", or "being substituted with one or two substituent groups".

In the present disclosure, the carbon number of a carbonyl group is not particularly limited, but is preferably 1 to 40. Specifically, it may be a substituent group having the following structure, but is not limited thereto.

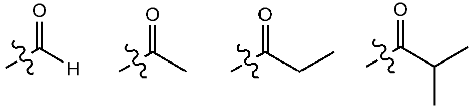

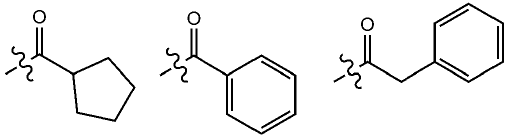

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent group having the following structural formulas, but is not limited thereto.

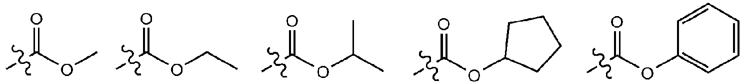

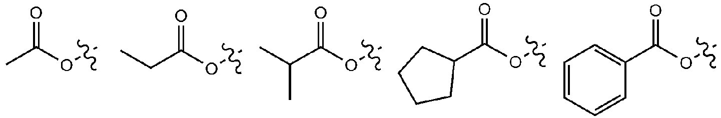

In the present disclosure, the carbon number of an imide group is not particularly limited, but is preferably 1 to 25. Specifically, the imide group may be a substituent group having the following structural formulas, but is not limited thereto.

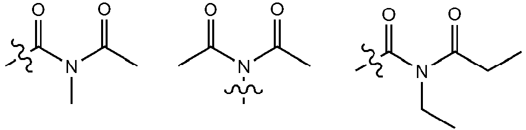

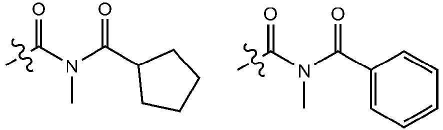

In the present disclosure, a substituted or unsubstituted silyl group refers to - Si(Z₁)(Z₂)(Z₃), wherein Z₁, Z₂ and Z₃ may each independently represent hydrogen, deuterium, a substituted or unsubstituted C₁₋₆₀ alkyl, a substituted or unsubstituted C₁₋₆₀ haloalkyl, a substituted or unsubstituted C₂₋₆₀ alkenyl, a substituted or unsubstituted C₂₋₆₀ haloalkenyl, or a substituted or unsubstituted C₆₋₆₀ aryl. According to one embodiment, Z₁, Z₂ and Z₃ may each independently represent hydrogen, deuterium, a substituted or unsubstituted C₁₋₁₀ alkyl, a substituted or unsubstituted C₁₋₁₀ haloalkyl, or a substituted or unsubstituted C₆₋₂₀ aryl. Specific examples of the silyl group includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present disclosure, a boron group specifically includes a trimethylboron group, a triethylboron group, a t-butyldimethylboron group, a triphenylboron group, a phenylboron group, and the like, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, specific examples of the alkyl group include methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, 1-ethylpropyl, 1,1-dimethylpropyl, hexyl, n-hexyl, 1-methylpentyl, 2-methylpentyl, 4-methyl-2-pentyl, 3,3-dimethylbutyl, 2-ethylbutyl, heptyl, n-heptyl, isohexyl, 1-methylhexyl, 2-methylhexyl, 3-methylhexyl, 4-methylhexyl, 5-methylhexyl, cyclopentylmethyl, cyclohexylmethyl, octyl, n-octyl, tert-octyl, 1-methylheptyl, 2-ethylhexyl, 2,4,4-trimethyl-1-pentyl, 2,4,4-trimethyl-2-pentyl, 2-propylpentyl, n-nonyl, 2,2-dimethylheptyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, the alicyclic group refers to a monovalent substituent group derived from a saturated or unsaturated hydrocarbon ring compound which contains only carbon as a ring-forming atom, but does not have aromaticity, and is understood to encompass both monocyclic or fused polycyclic compounds. According to one embodiment, the carbon number of the alicyclic group is 3 to 60. According to another embodiment, the carbon number of the cycloalkyl group is 3 to 30. According to still another embodiment, the carbon number of the cycloalkyl group is 3 to 20. Examples of such an alicyclic group include monocyclic groups such as a cycloalkyl group, bridged hydrocarbon groups, spirocyclic hydrocarbon groups, substituent groups derived from a hydrogenated derivative of an aromatic hydrocarbon compound, and the like.

Specific examples of the cycloalkyl group include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

Further, examples of the bridged hydrocarbon group include bicyclo[1.1.0]butyl, bicyclo[2.2.1]heptyl, bicyclo[4.2.0]octa-1,3,5-trienyl, adamantyl, decalinyl, and the like, but are not limited thereto.

Examples of the spirocyclic hydrocarbon group include spiro[3.4]octyl, spiro[5.5]undecanyl, and the like, but are not limited thereto.

Further, the substituent group derived from a hydrogenated derivative of an aromatic hydrocarbon compound refers to a substituent group derived from a compound in which hydrogen is added to a portion of the unsaturated bond of a monocyclic or polycyclic aromatic hydrocarbon compound. Examples of such a substituent group include 1*H*-indenyl, 2*H*-indenyl, 4*H*-indenyl, 2,3-dihydro-1*H*-indenyl, 1,4-dihydronaphthalenyl, 1,2,3,4-tetrahydronaphthalenyl, 6,7,8,9-tetrahydro-5*H-*benzo[7]annulenyl, 6,7-dihydro-5*H*-benzocycloheptenyl, and the like, but are not limited thereto.

In the present disclosure, an aryl group is understood to mean a substituent group derived from a monocyclic or fused polycyclic compound having aromaticity while containing only a carbon atom as a ring-constituting atom, and the carbon number thereof is not particularly limited, but is preferably 6 to 60. According to one embodiment, the carbon number of the aryl group is 6 to 30. According to one embodiment, the carbon number of the aryl group is 6 to 20. The aryl group may be a phenyl group, a biphenylyl group, a terphenylyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto.

In the present disclosure, the fluorenyl group may be substituted, and two substituent groups may be linked with each other to form a spiro structure. In the case where the fluorenyl group is substituted, 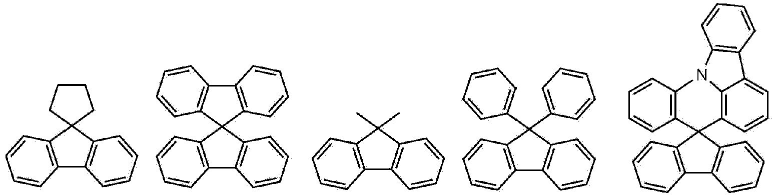 and the like can be formed. However, the structure is not limited thereto.

In the present disclosure, a heterocyclic group refers to a monovalent substituent group derived from a monocyclic or fused polycyclic compound further containing at least one heteroatom selected from O, N, Si and S in addition to a carbon atom as a ring-constituting atom, and is understood to encompass both a substituent group having aromaticity and a substituent group having no aromaticity. According to one embodiment, the carbon number of the heterocyclic group is 2 to 60. According to another embodiment, the carbon number of the heterocyclic group is 2 to 30. According to yet another embodiment, the carbon number of the heterocyclic group is 2 to 20. Examples of the heterocyclic group include a heteroaryl group, a substituent group derived from a hydrogenated derivative of a heteroaromatic compound, and the like.

Specifically, a heteroaryl group refers to a substituent group derived from a monocyclic or fused polycyclic compound further containing at least one heteroatom selected from N, O and S in addition to a carbon atom as a ring-constituting atom, and means a substituent group having aromaticity. According to one embodiment, the carbon number of the heteroaryl group is 2 to 60. According to another embodiment, the carbon number of the heteroaryl group is 2 to 30. According to another embodiment, the carbon number of the heteroaryl group is 2 to 20. Examples of the heteroaryl group include a thiophenyl group, a furanyl group, a pyrrolyl group, an imidazolyl group, a thiazolyl group, an oxazolyl group, an oxadiazolyl group, a triazolyl group, a pyridinyl group, a bipyridinyl group, a pyrimidinyl group, a triazinyl group, an acridinyl group, a pyridazinyl group, a pyrazinyl group, a quinolinyl group, a quinazolinyl group, a quinoxalinyl group, a phthalazinyl group, a pyridopyrimidinyl group, a pyridopyrazinyl group, an isoquinolinyl group, an indolyl group, a carbazolyl group, a benzoxazolyl group, a benzoimidazolyl group, a benzothiazolyl group, a benzocarbazolyl group, a benzothiophenyl group, a dibenzothiophenyl group, a benzofuranyl group, a dibenzofuranyl group, a phenanthrolinyl group, an isoxazolyl group, a thiadiazolyl group, a phenothiazinyl group, and the like, but are not limited thereto.

Further, the substituent group derived from a hydrogenated derivative of a heteroaromatic compound refers to a substituent group derived from a compound in which hydrogen is added to a portion of the unsaturated bond of a monocyclic or polycyclic heteroaromatic compound. Examples of such a substituent group include 1,3-dihydroisobenzofuranyl, 2,3-dihydrobenzofuranyl, 1,3-dihydrobenzo[c]thiophenyl, 2,3-dihydro[*b*]thiophenyl (2,3-dihydro[*b*]thiophenyl), and the like, but are not limited thereto.

In the present disclosure, the aryl group in the aralkyl group, the aralkenyl group, the alkylaryl group, the arylamine group and the arylsilyl group is the same as the examples of the aryl group as defined above. In the present disclosure, the alkyl group in the aralkyl group, the alkylaryl group and the alkylamine group is the same as the examples of the alkyl group as defined above. In the present disclosure, the heteroaryl in the heteroarylamine can be applied to the description of the heteroaryl as defined above. In the present disclosure, the alkenyl group in the aralkenyl group is the same as the examples of the alkenyl group as defined above. In the present disclosure, the description of the aryl group as defined above may be applied except that the arylene is a divalent group. In the present disclosure, the description of the heteroaryl as defined above can be applied except that the heteroarylene is a divalent group. In the present disclosure, the description of the aryl group or cycloalkyl group as defined above can be applied except that the hydrocarbon ring is not a monovalent group but formed by combining two substituent groups. In the present disclosure, the description of the heteroaryl as defined above can be applied, except that the heterocycle is not a monovalent group but formed by combining two substituent groups.

In the present disclosure, the term "deuterated or substituted with deuterium" means that at least one of the substitutable hydrogens in a compound, a divalent linking group, or a monovalent substituent group has been substituted with deuterium.

Further, the term "unsubstituted or substituted with deuterium" or "substituted or unsubstituted with deuterium" means that "mono to the maximum number of unsubstituted or substitutable hydrogens have been substituted with deuterium." In one example, the term "phenanthryl unsubstituted or substituted with deuterium" may be understood to mean "phenanthryl unsubstituted or substituted with 1 to 9 deuterium atoms", considering that the maximum number of hydrogen that can be substituted with deuterium in the phenanthryl structure is 9.

Further, the term "deuterated structure" is meant to encompass a compound, a divalent linking group or a monovalent substituent of all structures in which at least one hydrogen is replaced with deuterium. In one example, a deuterated structure of phenyl may be understood to refer to monovalent substituents of all structures in which at least one substitutable hydrogen in a phenyl group is replaced with a deuterium as follows.

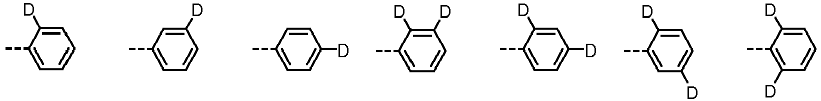

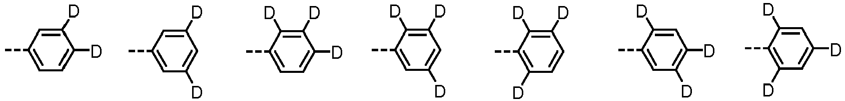

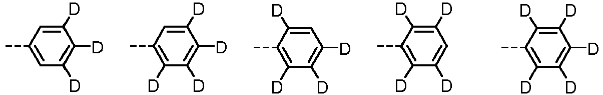

In addition, the "deuterium substitution rate" or "degree of deuteration" of a compound means that the ratio of the number of substituted deuterium atoms to the total number of hydrogen atoms (the sum of the number of hydrogen atoms substitutable with deuterium and the number of substituted deuterium atoms in a compound) that can be present in the compound is calculated as a percentage. Therefore, when the "deuterium substitution rate" or "degree of deuteration" of a compound is "K%", it means that K% of the hydrogen atoms substitutable with deuterium in the compound are substituted with deuterium.

At this time, the "deuterium substitution rate" or "degree of deuteration" can be measured according to a commonly known method using MALDI-TOF MS (Matrix-Assisted Laser Desorption/Ionization Time-of-Flight Mass Spectrometer), a nuclear magnetic resonance spectroscopy (¹H NMR), TLC/MS (Thin-Layer Chromatography/Mass Spectrometry), GC/MS (Gas Chromatography/Mass Spectrometry), or the like. More specifically, when using MALDI-TOF MS, the "deuterium substitution rate" or "degree of deuteration" is obtained by determining the number of substituted deuterium atoms in the compound through MALDI-TOF MS analysis, and then calculating the ratio of the number of substituted deuterium atoms to the total number of hydrogen atoms that can be present in the compound as a percentage.

### (Compound)

According to the present disclosure, there is provided the compound represented by Chemical Formula 1.

Preferably, the Chemical Formula 1 is represented by any one of the following Chemical Formulas 1-1 to 1-3: 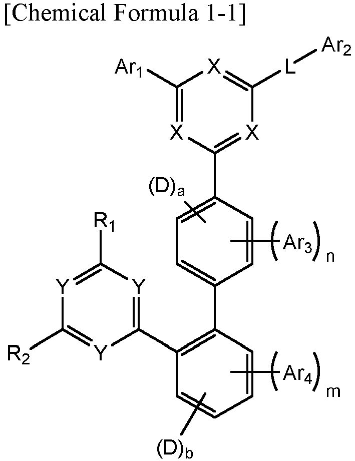 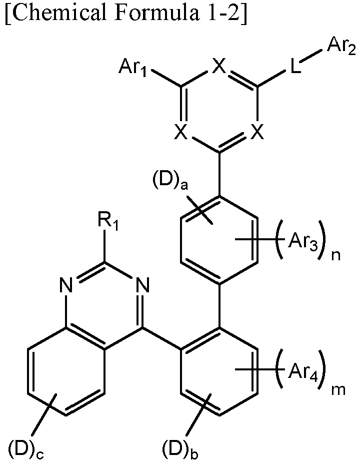 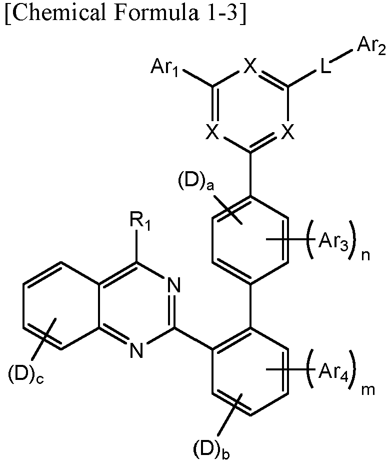 wherein, in Chemical Formulas 1-1 to 1-3,
X, L, Ar₁, Ar₂, Ar₃, Ar₄, a, b, n and m are as defined above,
Y is N or CR', with the proviso that two or more of the Y are N,
R' is hydrogen, deuterium, a substituted or unsubstituted C₁₋₆₀ alkyl, or a substituted or unsubstituted C₆₋₆₀ aryl,
R₁ and R₂ are each independently a substituted or unsubstituted C₆₋₆₀ aryl, and
c is an integer from 0 to 4.

Preferably, the Chemical Formula 1 is represented by any one of the following Chemical Formulas 1-4 to 1-9: 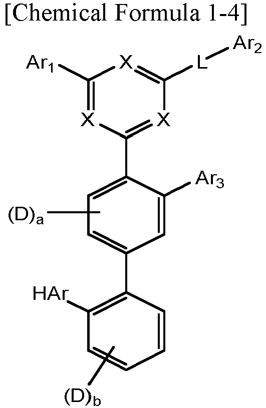 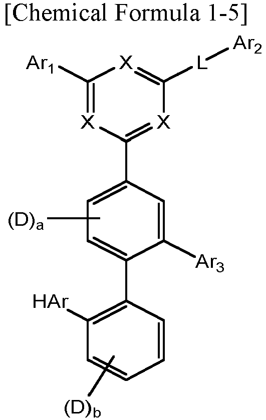 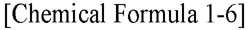 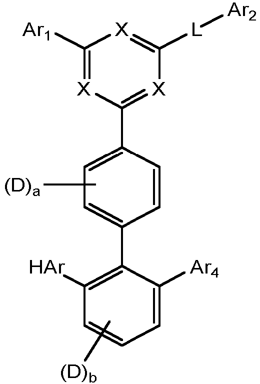 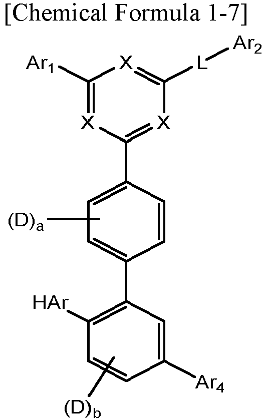 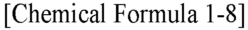 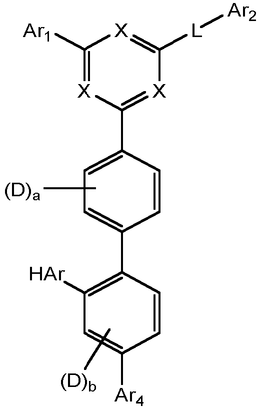 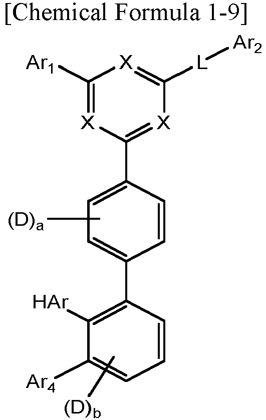 wherein, in Chemical Formulas 1-4 to 1-9,
X, L, Ar₁, Ar₂, Ar₃, Ar₄, HAr, a, and b are as defined above.

Preferably, L is a single bond, phenylene, or biphenyldiyl, wherein the phenylene and biphenyldiyl are unsubstituted or substituted with at least one deuterium.

Preferably, Ar₁ is phenyl unsubstituted or substituted with at least one deuterium.

Preferably, Ar₂ is phenyl, biphenylyl, or naphthyl, wherein the Ar₂ is unsubstituted or substituted with at least one deuterium.

Preferably, L is phenylene or biphenyldiyl, wherein the L is unsubstituted or substituted with at least one deuterium, and
Ar₂ is phenyl substituted with cyano, or naphthyl substituted with cyano, wherein the Ar₂ is substituted or unsubstituted with deuterium.

More preferably, L is phenylene unsubstituted or substituted with at least one deuterium, or biphenyldiyl unsubstituted or substituted with at least one deuterium; and Ar₂ is phenyl substituted with one cyano and 0 to 4 deuterium, or naphthyl substituted with one cyano and 0 to 6 deuterium.

Preferably, L is a single bond; Ar₂ is phenyl or biphenylyl, wherein the Ar₂ is unsubstituted or substituted with at least one deuterium, and
Ar₃ is phenyl substituted with cyano, wherein the Ar₃ is substituted or unsubstituted with deuterium.

More preferably, L is a single bond; Ar₂ is phenyl unsubstituted or substituted with at least one deuterium, or biphenylyl unsubstituted or substituted with at least one deuterium; and Ar₃ is phenyl substituted with one cyano and 0 to 4 deuterium.

Preferably, R' is hydrogen, deuterium, or methyl unsubstituted or substituted with at least one deuterium.

Preferably, R₁ and R₂ are each independently phenyl, biphenylyl, or naphthyl, wherein the R₁ and R₂ are unsubstituted or substituted with at least one deuterium.

Representative examples of the compound represented by Chemical Formula 1 is as follows:

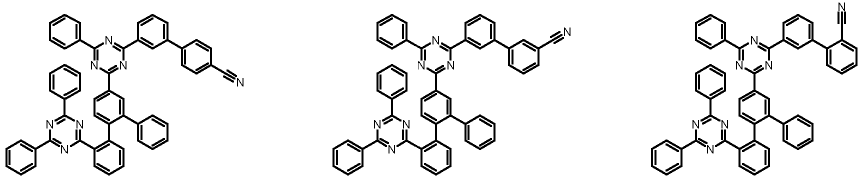

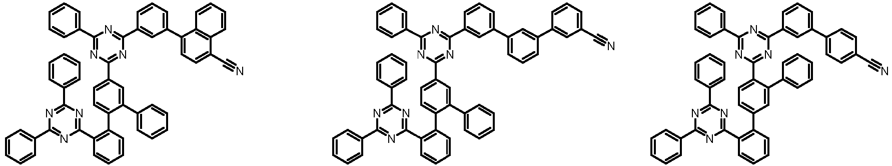

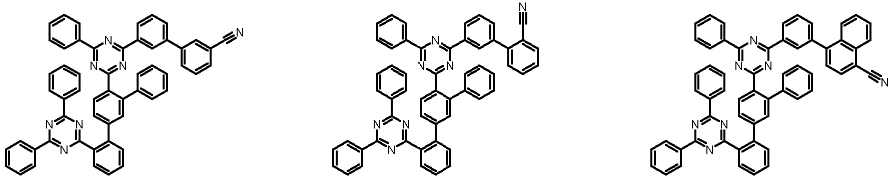

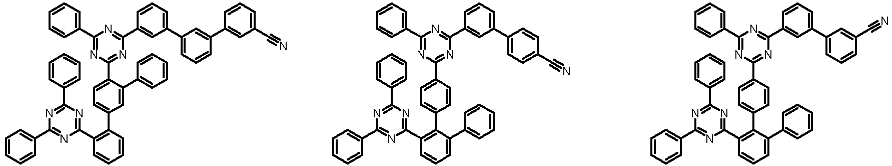

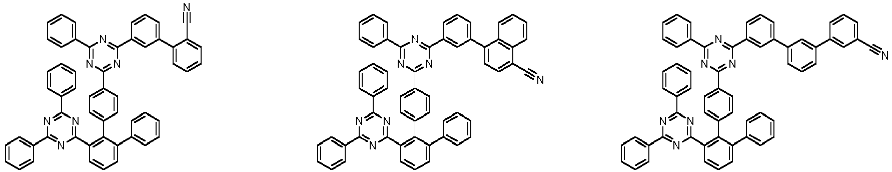

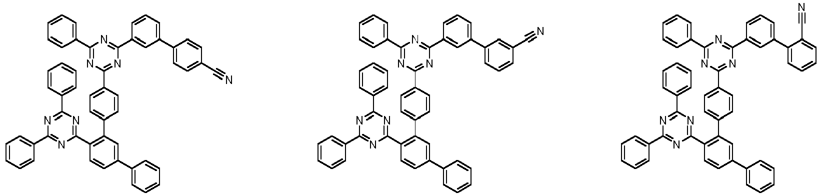

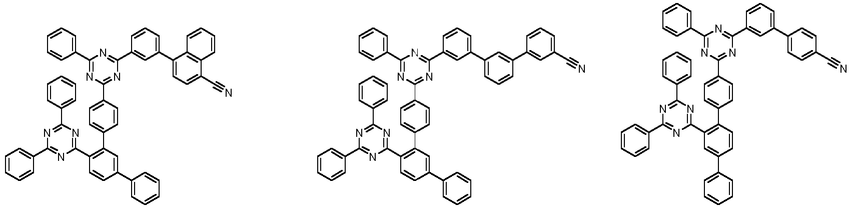

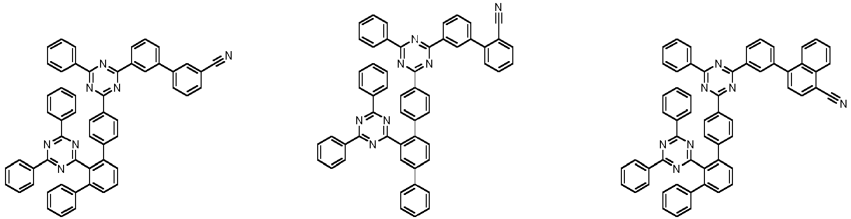

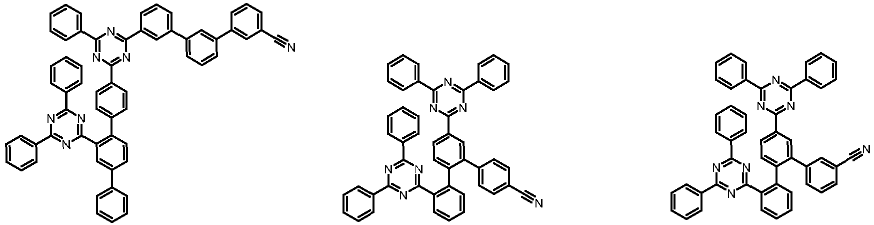

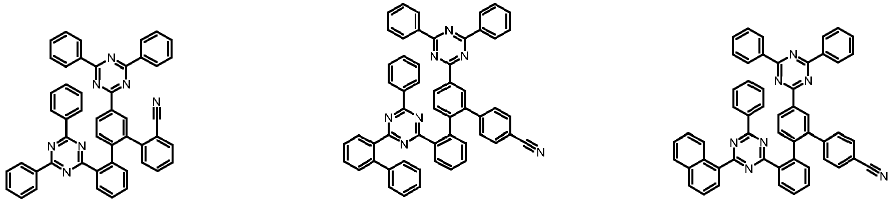

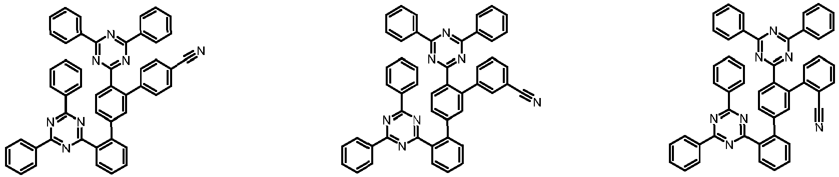

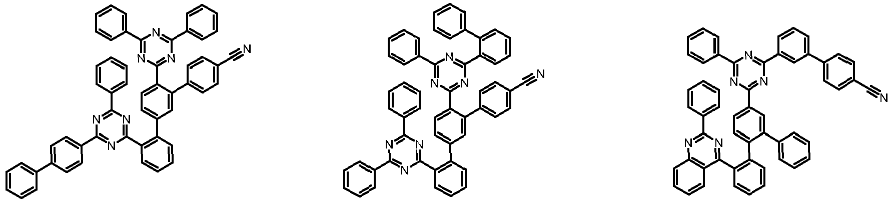

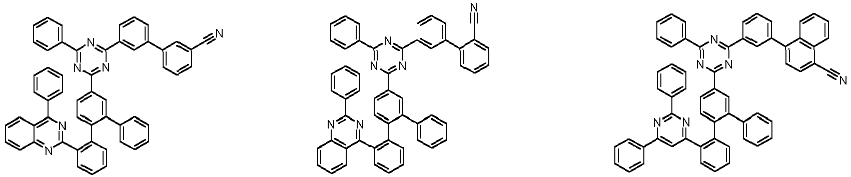

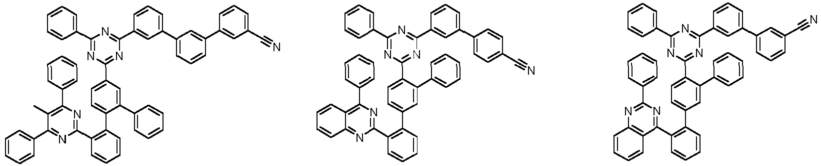

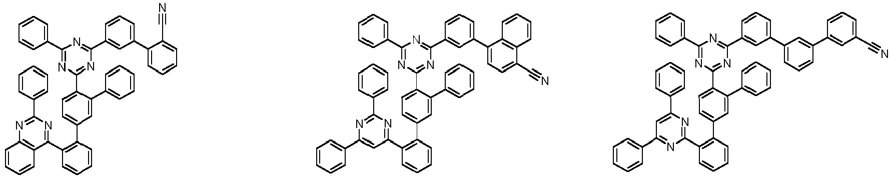

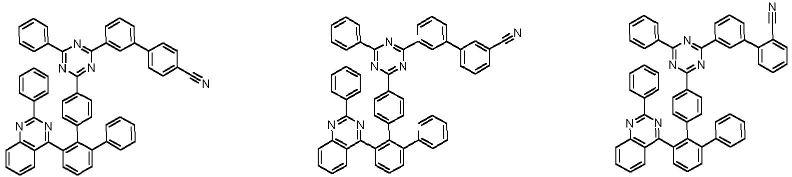

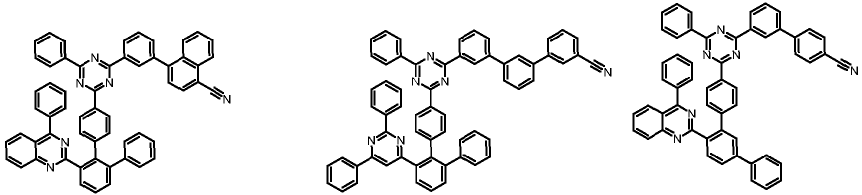

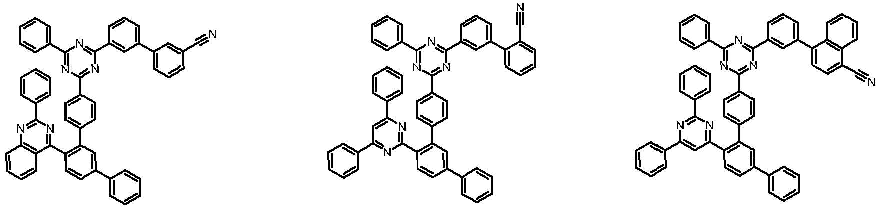

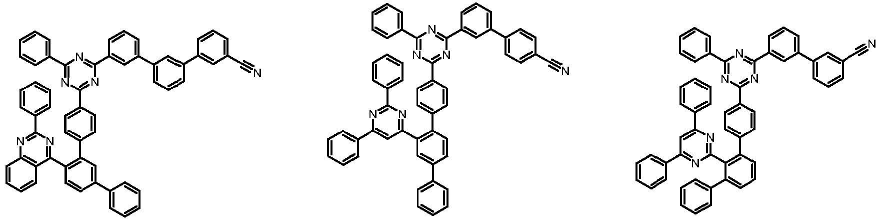

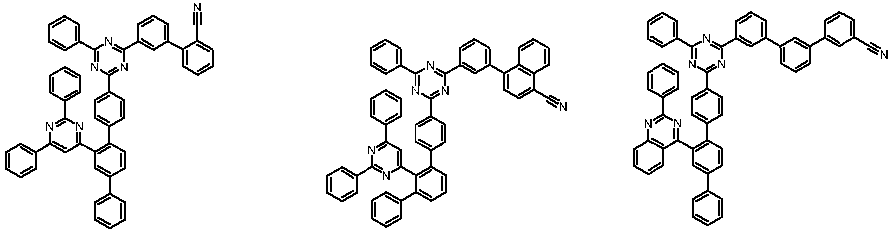

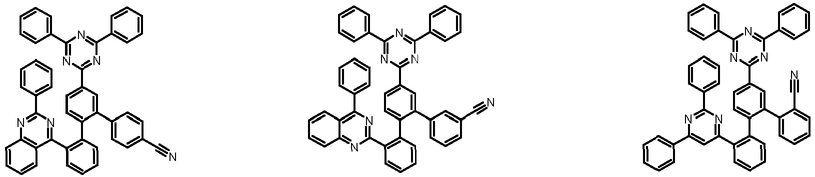

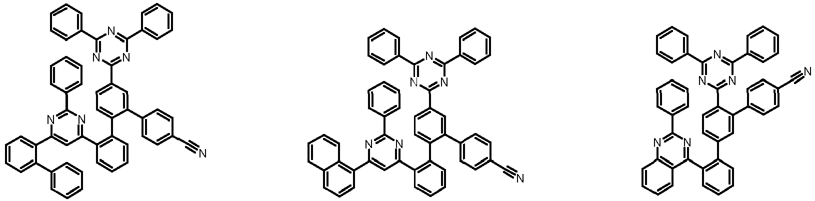

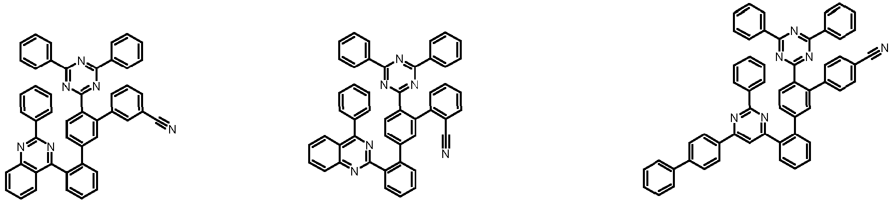

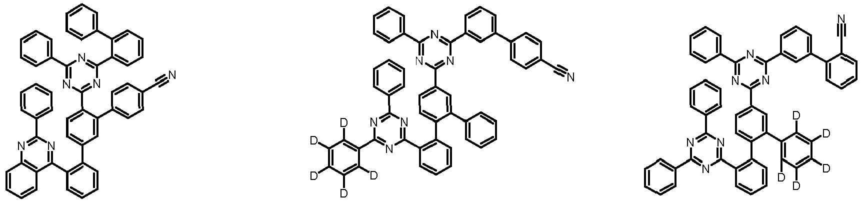

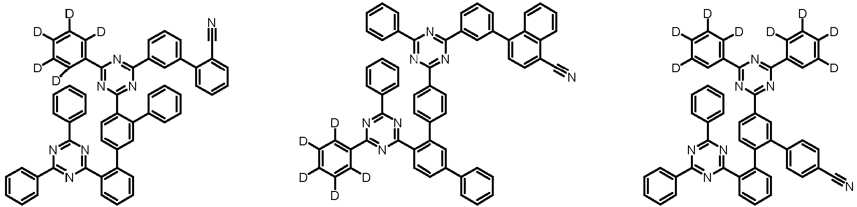

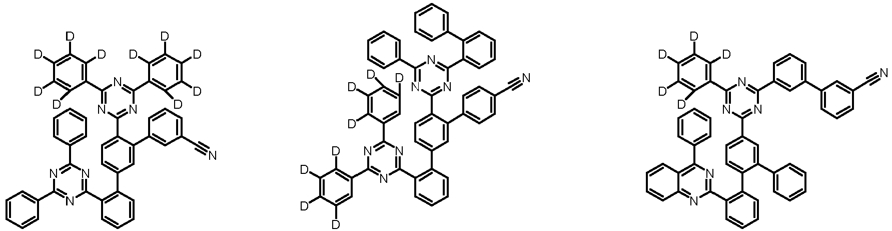

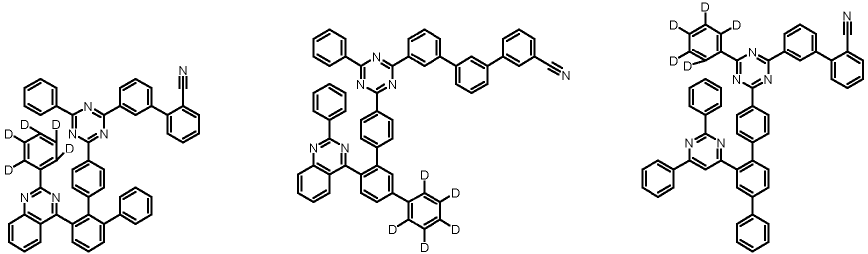

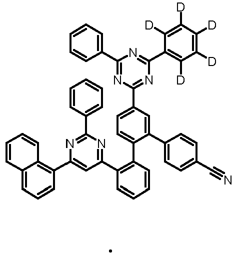

Additionally, according to the present disclosure, there is provided a method for preparing the compound represented by Chemical Formula 1 as shown in the following Reaction Scheme 1. 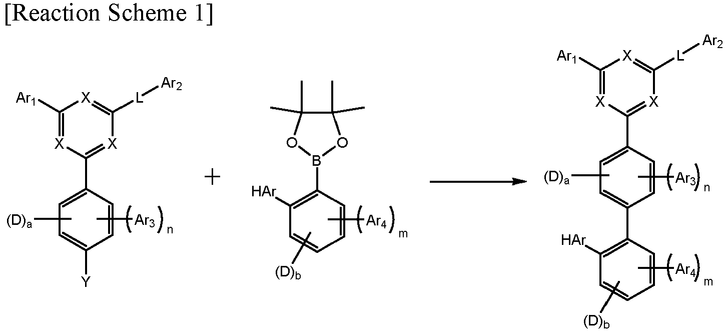 in Reaction Scheme 1, X, L, Ar₁, Ar₂, Ar₃, Ar₄, HAr, a, b, n and m are the same as defined above, and Y is halogen, preferably bromo or chloro.

The compound represented by Chemical Formula 1 can be prepared through a Suzuki coupling reaction. At this time, the Suzuki coupling reaction is preferably carried out in the presence of a palladium catalyst and a base, and a reactive group for the reaction can be modified to any reactive group known in the art. Such a preparation method can be further embodied in Preparation Examples described later.

### (Organic Light Emitting Device)

Further, according to the present disclosure, there is provided an organic light emitting device comprising a compound represented by Chemical Formula 1. In one example, the present disclosure provides an organic light emitting device comprising: a first electrode; a second electrode that is provided opposite to the first electrode; and one or more organic material layers that are provided between the first electrode and the second electrode, wherein at least one layer of the organic material layers includes the compound represented by Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, an electron blocking layer, a light emitting layer, an electron transport layer, an electron injection layer and the like as the organic material layer. However, the structure of the organic light emitting device is not limited thereto, and it may include a smaller number of organic layers.

Further, the organic material layer may include a light emitting layer, wherein the light emitting layer may include the compound represented by Chemical Formula 1. Particularly, the compound according to the present disclosure can be used as a dopant of the light emitting layer.

Further, the organic material layer may include an electron transport layer, an electron injection layer, or a layer for simultaneously performing electron transport and electron injection, wherein the electron transport layer, the electron injection layer, and the layer for simultaneously performing electron transport and electron injection may include the compound represented by Chemical Formula 1.

Further, the organic material layer may include , a hole injection layer, a hole transport layer, a light emitting layer, or an electron transport and injection layer, wherein the organic material layer including the above compound may be an electron transport and injection layer.

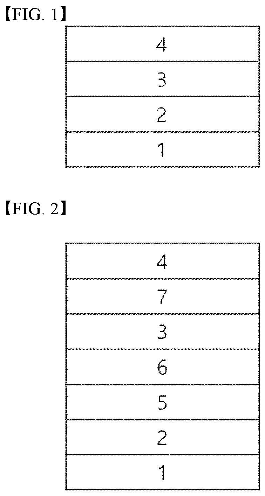

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of the organic light emitting device according to an embodiment of the present disclosure is illustrated in Figs. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a light emitting layer 3, an electron transport and injection layer 7, and a cathode 4. In such a structure, the compound represented by Chemical Formula 1 may be included in the electron transport and injection layer.

The organic light emitting device according to the present disclosure may be manufactured by materials and methods known in the art, except that the light emitting layer includes the compound according to the present disclosure and is manufactured as described above. Further, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device can be manufactured by sequentially depositing a cathode material, an organic material layer and an anode material on a substrate.

Further, the compound represented by Chemical Formula 1 can be formed into an organic layer by a solution coating method as well as a vacuum deposition method at the time of manufacturing an organic light emitting device. Wherein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to a electron injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxy-quinoline aluminum complex (Alq₃); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, lubrene, and the like, but are not limited thereto.

The light emitting layer may include a host material and a dopant material. The host material includes a fused aromatic ring derivative, a heterocycle-containing compound, or the like. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

Examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

The electron injection and transport layer is a layer for simultaneously performing the roles of an electron transport layer and an electron injection layer that inject electrons from an electrode and transport the received electrons up to the light emitting layer, and is formed on the light emitting layer or the hole blocking layer. The electron injection and transport material is suitably a material which can receive electrons well from a cathode and transfer the electrons to a light emitting layer, and has a large mobility for electrons. The compound represented by Chemical Formula 1 can be used as the electron injection and transport layer material. Further examples of the electron injection and transport material includes: an Al complex of 8-hydroxyquinoline; a complex including Alq₃; an organic radical compound; a hydroxyflavone-metal complex, a triazine derivative, and the like, but are not limited thereto. Alternatively, it may be used together with fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

The electron injection and transport layer may also be formed as a separate layer such as an electron injection layer and an electron transport layer. In such a case, the electron transport layer is formed on the light emitting layer or the hole blocking layer, and the above-mentioned electron injection and transport material may be used as the electron transport material included in the electron transport layer. In addition, the electron injection layer is formed on the electron transport layer, and examples of the electron injection material included in the electron injection layer include LiF, NaCl, CsF, Li₂O, BaO, fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like.

The metal complex compound includes 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but is not limited thereto.

In one embodiment, the electron transport layer, the electron injection layer, or the electron injection and transport layer of the organic light emitting device may include the compound represented by Chemical Formula 1 and the metal complex compound. In this case, the electron transport layer, the electron injection layer, or the electron injection and transport layer may contain the compound represented by Chemical Formula 1 and the metal complex compound in a weight ratio of 10:90 to 90:10 and a weight ratio of 30:70 to 70 :30, or a weight ratio of 50:50.

The organic light emitting device according to the present disclosure may be a bottom emission type device, a top emission type device, or a double side emission type device, and in particular, it may be a bottom emission type light emitting device that requires relatively high luminous efficiency.

In addition, the compound represented by Chemical Formula 1 may be included in an organic solar cell or an organic transistor in addition to an organic light emitting device.

The preparation of the compound represented by Chemical Formula 1 and the organic light emitting device including the same will be specifically described in the following Examples. However, the following Examples are provided for illustrative purposes only, and are not intended to limit the scope of the present disclosure.

### [EXAMPLE]

### Example 1: Preparation of Compound E1

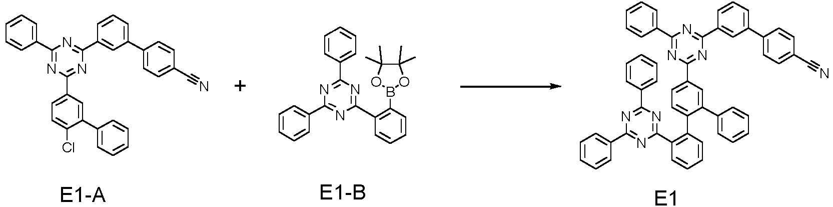

E1-A (20 g, 38.4 mmol) and E1-B (16.7 g, 38.4 mmol) were added to 400 ml of 1,4-dioxane under a nitrogen atmosphere, and the mixture was stirred and refluxed. Then, potassium phosphate tribasic (24.4 g, 115.2 mmol) was dissolved in 24 ml of water and added thereto, and the mixture was sufficiently stirred, and then dibenzylideneacetonepalladium (0.7 g, 1.2 mmol) and tricyclohexylphosphine (0.6 g, 2.3 mmol) were added thereto. After the reaction for 7 hours, the reaction mixture was cooled to room temperature, and then the produced solid was filtered. The solid was added to 914 mL of chloroform, dissolved, washed twice with water, and the organic layer was separated, to which anhydrous magnesium sulfate was added, stirred, then filtered, and the filtrate was distilled under reduced pressure. The concentrated compound was recrystallized from chloroform and ethyl acetate to give Compound E1 as a white solid (4.6 g, yield: 15%).

MS: [M+H]⁺ = 794

### Example 2: Preparation of Compound E2

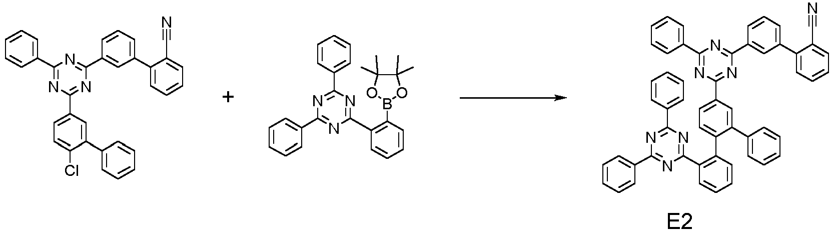

Compound E2 was prepared by the same process as the preparation method of Example 1, except that each starting material was used as in the above Reaction Scheme.

MS: [M+H]⁺ = 794

### Example 3: Preparation of Compound E3

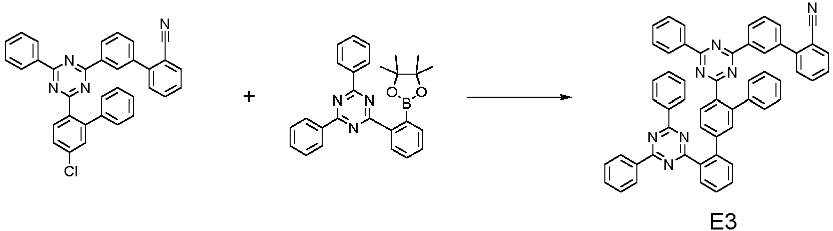

Compound E3 was prepared by the same process as the preparation method of Example 1, except that each starting material was used as in the above Reaction Scheme.

MS: [M+H]⁺ = 794

### Example 4: Preparation of Compound E4

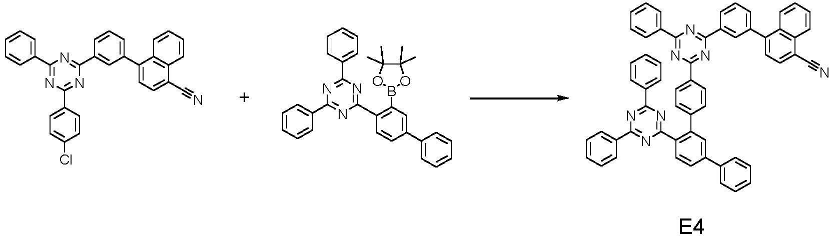

Compound E4 was prepared by the same process as the preparation method of Example 1, except that each starting material was used as in the above Reaction Scheme.

MS: [M+H]⁺ = 844

### Example 5: Preparation of Compound E5

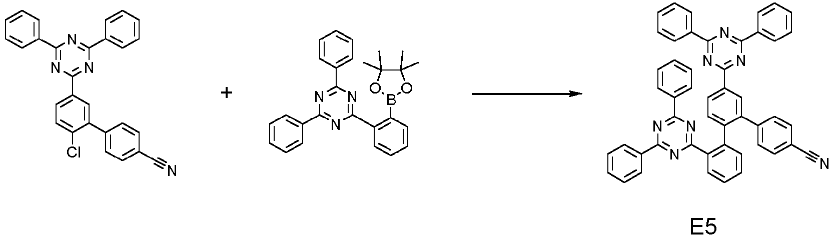

Compound E5 was prepared by the same process as the preparation method of Example 1, except that each starting material was used as in the above Reaction Scheme.

MS: [M+H]⁺ = 718

### Example 6: Preparation of Compound E6

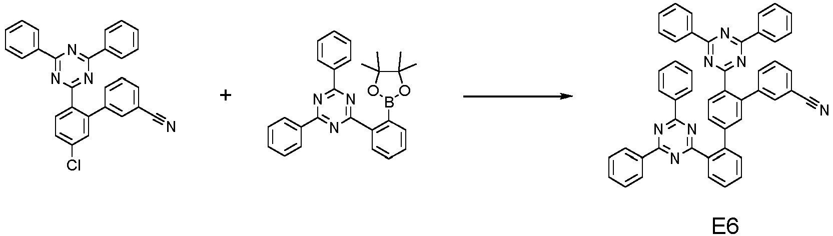

Compound E6 was prepared by the same process as the preparation method of Example 1, except that each starting material was used as in the above Reaction Scheme.

MS: [M+H]⁺ = 718

### Example 7: Preparation of Compound E7

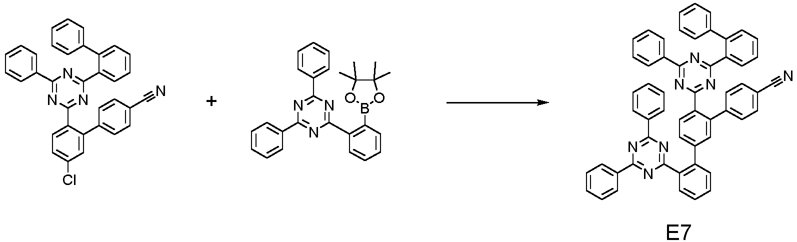

Compound E7 was prepared by the same process as the preparation method of Example 1, except that each starting material was used as in the above Reaction Scheme.

MS: [M+H]⁺ = 794

### Example 8: Preparation of Compound E8

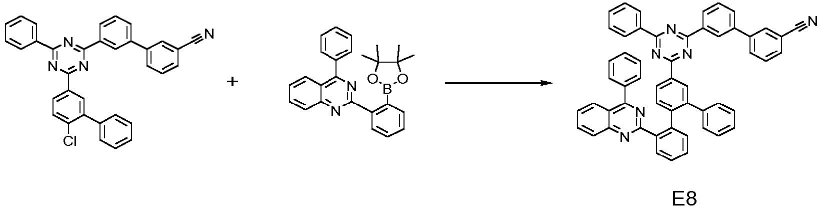

Compound E8 was prepared by the same process as the preparation method of Example 1, except that each starting material was used as in the above Reaction Scheme.

MS: [M+H]⁺ = 767

### Example 9: Preparation of Compound E9

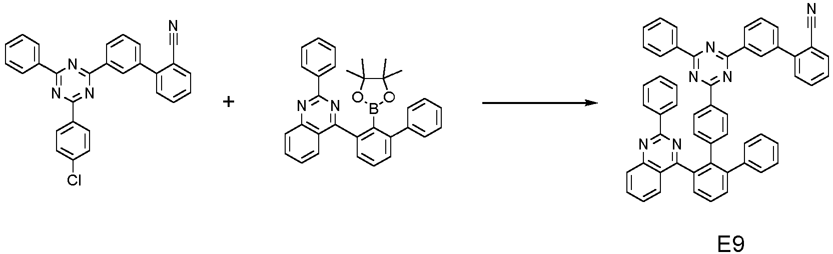

Compound E9 was prepared by the same process as the preparation method of Example 1, except that each starting material was used as in the above Reaction Scheme.

MS: [M+H]⁺ = 767

### Example 10: Preparation of Compound E10

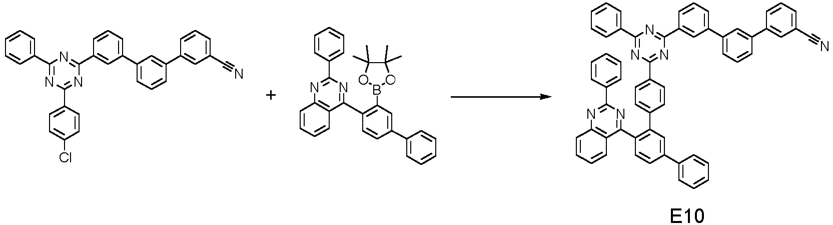

Compound E10 was prepared by the same process as the preparation method of Example 1, except that each starting material was used as in the above Reaction Scheme.

MS: [M+H]⁺ = 843

### Example 11: Preparation of Compound E11

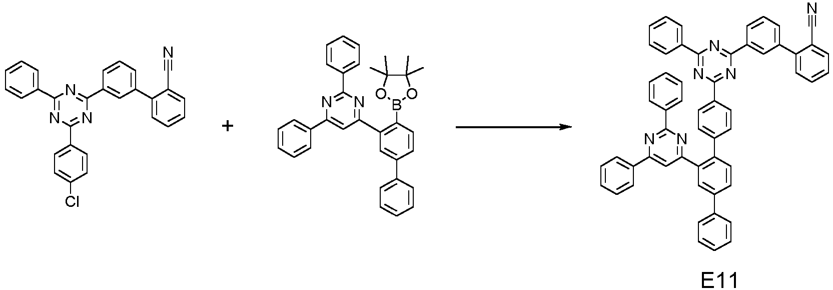

Compound E11 was prepared by the same process as the preparation method of Example 1, except that each starting material was used as in the above Reaction Scheme.

MS: [M+H]⁺ = 793

### Example 12: Preparation of Compound E12

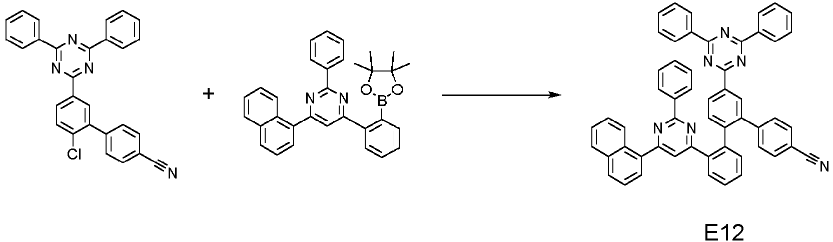

Compound E12 was prepared by the same process as the preparation method of Example 1, except that each starting material was used as in the above Reaction Scheme.

MS: [M+H]⁺ = 767

### Example 13: Preparation of Compound E13

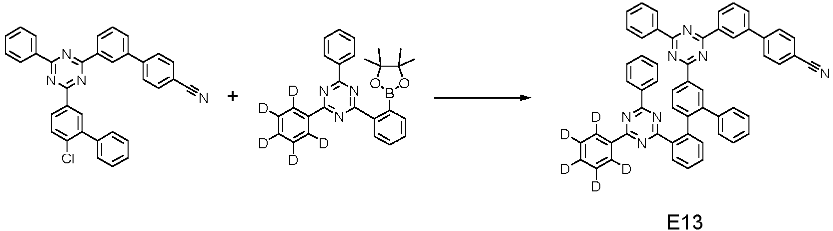

Compound E13 was prepared by the same process as the preparation method of Example 1, except that each starting material was used as in the above Reaction Scheme.

MS: [M+H]⁺ = 799

### [EXPERIMENTAL EXAMPLE]

### Experimental Example 1

A glass substrate on which a thin film of ITO (indium tin oxide) was coated in a thickness of 1,000 Å was put into distilled water containing the detergent dissolved therein, and ultrasonically washed. At this time, the used detergent was a product commercially available from Fisher Co. and the distilled water was one which had been twice filtered by using a filter commercially available from Millipore Co. After the ITO was washed for 30 minutes, ultrasonic washing was conducted twice repeatedly using distilled water for 10 minutes. After the washing using distilled water was completed, the substrate was ultrasonically washed with the solvents of isopropyl alcohol, acetone, and methanol, and dried, after which it was transported to a plasma cleaner. Then, the substrate was cleaned with oxygen plasma for 5 minutes, and then transferred to a vacuum evaporator.

On the ITO transparent electrode thus prepared, the following Compound HI-A was thermally vacuum-deposited to a thickness of 600 Å to form a hole injection layer. Hexaazatriphenylene hexanitrile (HAT, 50 Å) having the following chemical formula and the following Compound HT-A (600 Å) were sequentially vacuum-deposited on the hole injection layer to form a hole transport layer.

Then, the following Compounds BH and BD were vacuum-deposited at a weight ratio of 25:1 on the hole transport layer to a film thickness of 200 Å to form a light emitting layer. Compound E1 prepared in Example 1 and the following Compound LiQ (lithiumquinolate) were vacuum-deposited at a weight ratio of 1:1 on the light emitting layer to form an electron transport and injection layer with a thickness of 360 Å. Lithium fluoride (LiF) and aluminum were sequentially deposited to have a thickness of 10Å and 1,000Å, respectively, on the electron transport and injection layer, thereby forming a cathode.

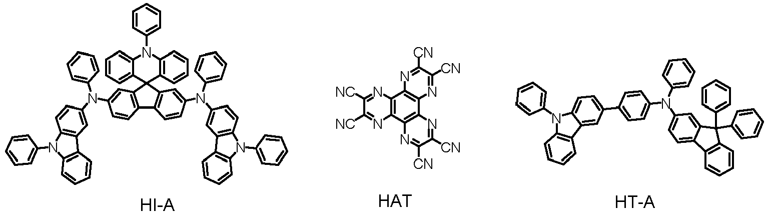

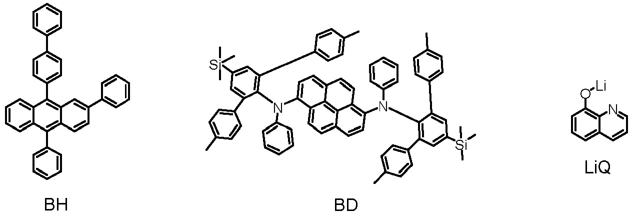

In the above-mentioned processes, the vapor deposition rate of the organic material was maintained at 0.4 ~ 0.9 Å/sec, the deposition rates of lithium fluoride and the aluminum of the cathode were maintained at 0.3 Å/sec and 2 Å/sec, respectively, and the degree of vacuum during the deposition was maintained at 1x10⁻⁷ ~ 5x10⁻⁸ torr, thereby manufacturing an organic light emitting device.

### Experimental Examples 2 to 13

The organic light emitting devices were manufactured in the same manner as in Experimental Example 1, except that the compounds shown in Table 1 below were used instead of Compound E1 of Experimental Example 1.

### Comparative Experimental Examples 1 to 13

The organic light emitting devices were manufactured in the same manner as in Experimental Example 1, except that the compounds shown in Table 1 below were used instead of Compound E1 of Experimental Example 1. The compounds ET-A to ET-M used in Table 1 below are as follows.

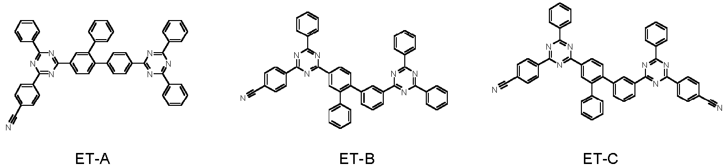

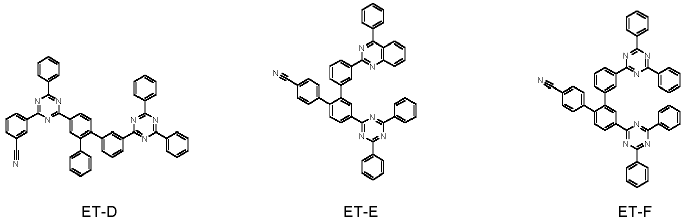

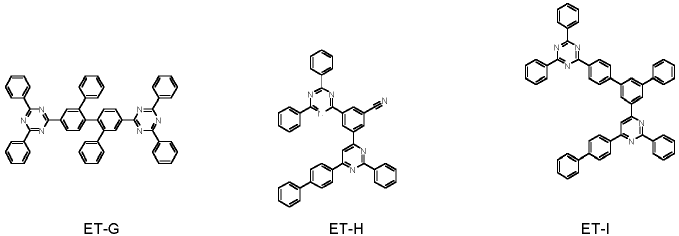

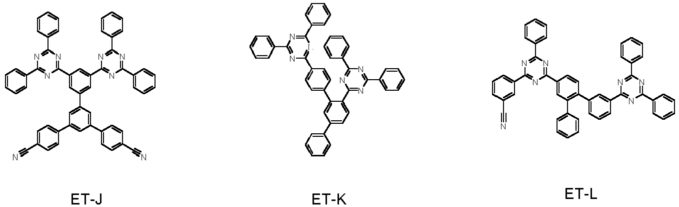

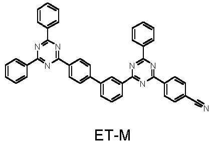

The driving voltage, luminous efficiency and color coordinate of the organic light emitting devices manufactured in the Experimental Examples and Comparative Experimental Examples were measured at a current density of 10 mA/cm², and the time (T90) required for the luminance to be reduced to 90% of the initial luminance was measured at a current density of 20 mA/cm². The results are shown in Table 1 below.

**[Table 1]**

| | Compound (electron transport and injection layer) | Voltage (V@10mA/cm²) | Efficiency (cd/A@10mA/cm ²) | Color coordinate (x,y) | T90 (hr@20mA/cm²) |
|---|---|---|---|---|---|
| Experimental Example 1 | E1 | 3.89 | 4.51 | (0.136, 0.112) | 201 |
| Experimental Example 2 | E2 | 3.85 | 4.61 | (0.136, 0.111) | 183 |
| Experimental Example 3 | E3 | 3.81 | 4.71 | (0.136, 0.112) | 166 |
| Experimental Example 4 | E4 | 3.97 | 4.42 | (0.136, 0.111) | 225 |
| Experimental Example 5 | E5 | 3.93 | 4.46 | (0.136, 0.111) | 219 |
| Experimental Example 6 | E6 | 3.89 | 4.56 | (0.136, 0.111) | 199 |
| Experimental Example 7 | E7 | 3.94 | 4.44 | (0.136, 0.112) | 221 |
| Experimental Example 8 | E8 | 4.01 | 4.24 | (0.136, 0.111) | 192 |
| Experimental Example 9 | E9 | 3.97 | 4.28 | (0.136, 0.112) | 175 |
| Experimental Example 10 | E10 | 3.93 | 4.32 | (0.136, 0.111) | 159 |
| Experimental Example 11 | E11 | 3.89 | 4.37 | (0.136, 0.111) | 145 |
| Experimental Example 12 | E12 | 3.85 | 4.41 | (0.136, 0.111) | 132 |
| Experimental Example 13 | E13 | 3.89 | 4.51 | (0.136, 0.112) | 220 |
| Comparative Experimental Example 1 | ET-A | 4.28 | 2.71 | (0.136, 0.111) | 121 |
| Comparative Experimental Example 2 | ET-B | 4.24 | 2.77 | (0.136, 0.111) | 110 |
| Comparative Experimental Example 3 | ET-C | 4.71 | 2.16 | (0.136, 0.112) | 133 |
| Comparative Experimental Example 4 | ET-D | 4.19 | 2.79 | (0.136, 0.111) | 99 |
| Comparative Experimental Example 5 | ET-E | 4.41 | 3.18 | (0.136, 0.112) | 127 |
| Comparative Experimental Example 6 | ET-F | 4.36 | 3.25 | (0.136, 0.111) | 115 |
| Comparative Experimental Example 7 | ET-G | 4.08 | 4.01 | (0.136, 0.111) | 20 |
| Comparative Experimental Example 8 | ET-H | 4.41 | 2.54 | (0.136, 0.112) | 115 |
| Comparative Experimental Example 9 | ET-I | 4.00 | 4.19 | (0.136, 0.111) | 17 |
| Comparative Experimental Example 10 | ET-J | 4.85 | 2.03 | (0.136, 0.111) | 127 |
| Comparative Experimental Example 11 | ET-K | 4.00 | 4.19 | (0.136, 0.112) | 19 |
| Comparative Experimental Example 12 | ET-L | 4.30 | 2.75 | (0.136, 0.111) | 90 |
| Comparative Experimental Example 13 | ET-M | 4.28 | 2.70 | (0.136, 0.111) | 85 |

As shown in Table 1, it was confirmed that the organic light emitting device using the compound represented by Chemical Formula 1 of the present disclosure exhibits excellent characteristics in terms of voltage, efficiency, and/or lifetime (T90).

Comparing Experimental Examples 1 to 13 and Comparative Experimental Examples 1, 2, 4 to 6, 8, 12 and 13 in Table 1, it was confirmed that the organic light emitting device containing the compound of Chemical Formula 1 of the present disclosure exhibits significantly superior characteristics in terms of efficiency than the organic light emitting device using a compound having different substituent groups between heterocyclic groups.

Comparing Experimental Examples 1 to 13 and Comparative Experimental Examples 3 and 10 in Table 1, it was confirmed that the organic light emitting device containing the compound of Chemical Formula 1 of the present disclosure exhibits significantly superior characteristics in terms of efficiency than the organic light emitting device using a compound in which two cyano groups were substituted.

Comparing Experimental Examples 1 to 13 and Comparative Experimental Examples 7, 9 and 11 in Table 1, it was confirmed that the organic light emitting device containing the compound of Chemical Formula 1 of the present disclosure exhibits significantly superior characteristics in terms of lifetime than the organic light emitting device using a compound in which a cyano group was unsubstituted.

### [Description of Symbols]

| | | | |
|---|---|---|---|
| 1: | substrate | 2: | anode |
| 3: | light emitting layer | 4: | cathode |
| 5: | hole injection layer | 6: | hole transport layer |
| 7: | electron transport and injection layer | | |

## Claims

1. A compound represented by the following Chemical Formula 1: 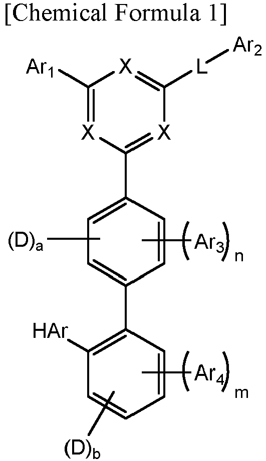 wherein, in Chemical Formula 1,
each X is independently N, CH, or CD, with the proviso that two or more of the X are N,
L is a single bond, or a substituted or unsubstituted C₆₋₆₀ arylene,
Ar₁ and Ar₂ are each independently a substituted or unsubstituted C₆₋₆₀ aryl,
Ar₃ and Ar₄ are each independently phenyl unsubstituted or substituted with at least one deuterium,
any one of Ar₁ to Ar₄ is substituted with cyano,
HAr is pyrimidinyl which is unsubstituted or substituted with 1 to 3 substituent groups each independently selected from the group consisting of deuterium, a substituted or unsubstituted C₁₋₆₀ alkyl, and a substituted or unsubstituted C₆₋₆₀ aryl; triazinyl which is substituted with two substituted or unsubstituted C₆₋₆₀ aryls; or quinazolinyl which is substituted with one substituted or unsubstituted C₆₋₆₀ aryl,
D means deuterium,
a and b are each independently an integer from 0 to 4,
n and m are each independently 0 or 1,
with the proviso that n+m is 1,
a+n is an integer from 0 to 4, and
b+m is an integer from 0 to 4.

2. The compound of claim 1, wherein:
the Chemical Formula 1 is represented by any one of the following Chemical Formulas 1-1 to 1-3: 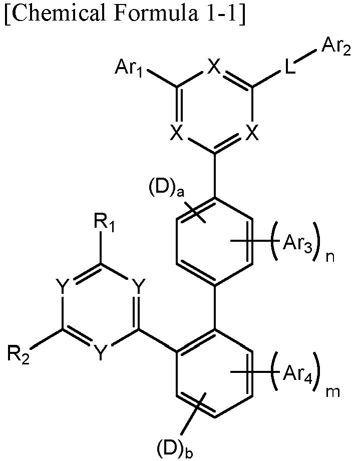 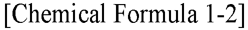 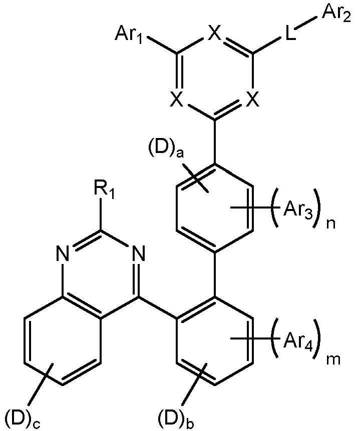 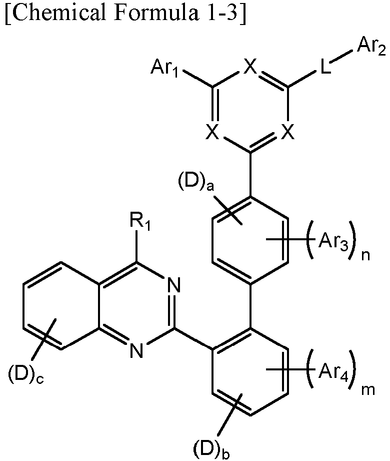
wherein, in Chemical Formulas 1-1 to 1-3,
X, L, Ar₁, Ar₂, Ar₃, Ar₄, D, a, b, n and m are as defined in claim 1,
Y is N or CR', with the proviso that two or more of the Y are N,
R' is hydrogen, deuterium, a substituted or unsubstituted C₁₋₆₀ alkyl, or a substituted or unsubstituted C₆₋₆₀ aryl,
R₁ and R₂ are each independently a substituted or unsubstituted C₆₋₆₀ aryl, and
c is an integer from 0 to 4.

3. The compound of claim 1, wherein:
the Chemical Formula 1 is represented by any one of the following Chemical Formulas 1-4 to 1-9: 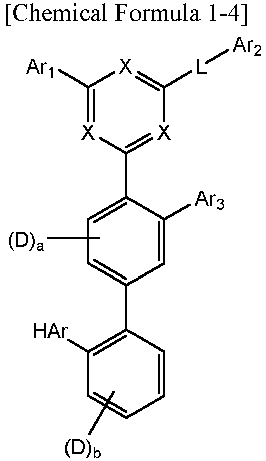 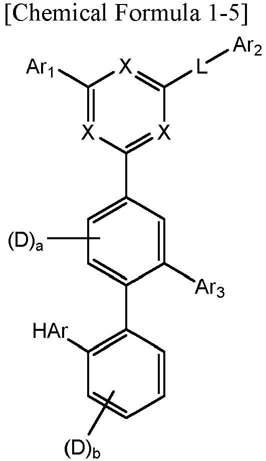 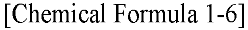 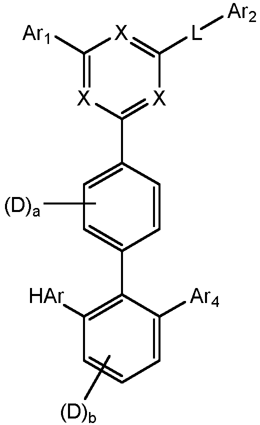 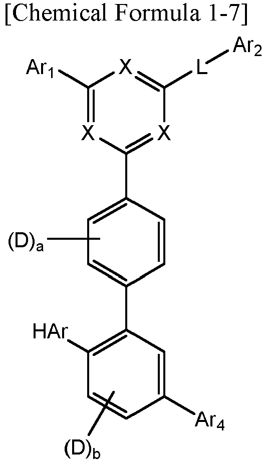 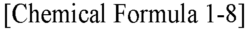 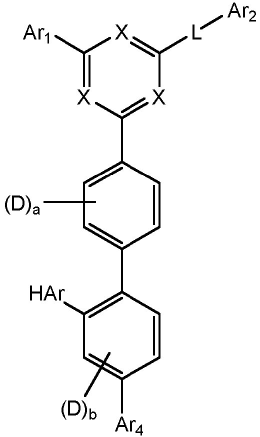 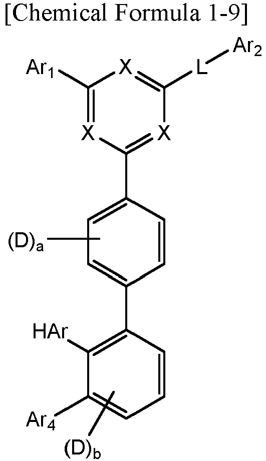
wherein, in Chemical Formulas 1-4 to 1-9,
X, L, Ar₁, Ar₂, Ar₃, Ar₄, HAr, D, a, and b are as defined in claim 1.

4. The compound of claim 1, wherein:
L is a single bond, phenylene, or biphenyldiyl,
wherein the phenylene and biphenyldiyl are unsubstituted or substituted with at least one deuterium.

5. The compound of claim 1, wherein:
Ar₁ is phenyl unsubstituted or substituted with at least one deuterium.

6. The compound of claim 1, wherein:
Ar₂ is phenyl, biphenylyl, or naphthyl,
wherein the Ar₂ is unsubstituted or substituted with at least one deuterium.

7. The compound of claim 1, wherein:
L is phenylene or biphenyldiyl,
wherein the L is unsubstituted or substituted with at least one deuterium, and
Ar₂ is phenyl substituted with cyano, or naphthyl substituted with cyano,
wherein the Ar₂ is substituted or unsubstituted with deuterium.

8. The compound of claim 1, wherein:
L is a single bond,
Ar₂ is phenyl or biphenylyl,
wherein the Ar₂ is unsubstituted or substituted with at least one deuterium, and
Ar₃ is phenyl substituted with cyano,
wherein the Ar₃ is substituted or unsubstituted with deuterium.

9. The compound of claim 2, wherein:
R' is hydrogen, deuterium, or methyl unsubstituted or substituted with at least one deuterium.

10. The compound of claim 2, wherein:
R₁ and R₂ are each independently phenyl, biphenylyl, or naphthyl,
wherein the R₁ and R₂ are unsubstituted or substituted with at least one deuterium.

11. The compound of claim 1, wherein:
the compound represented by Chemical Formula 1 is any one selected from the group consisting of compounds of the following structural formulas:

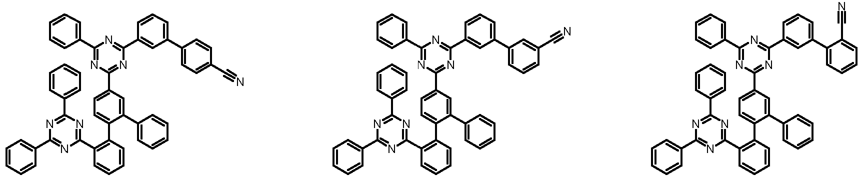

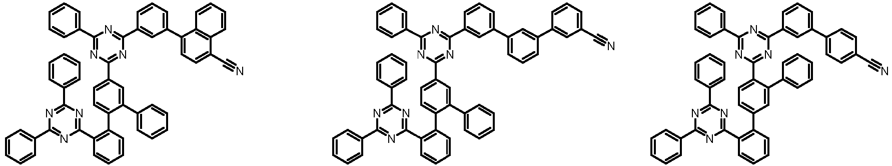

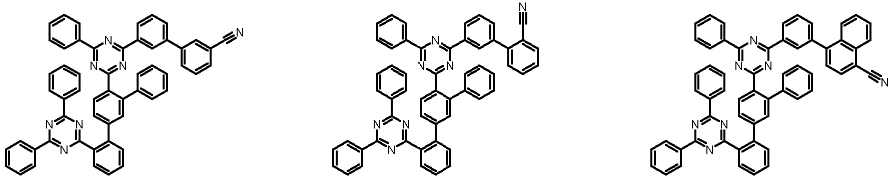

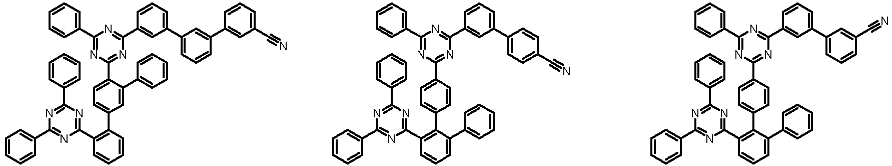

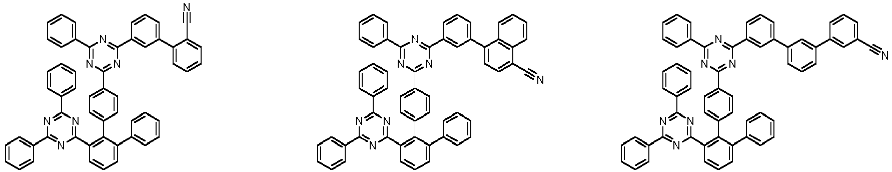

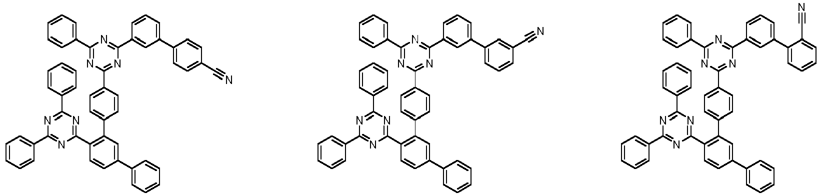

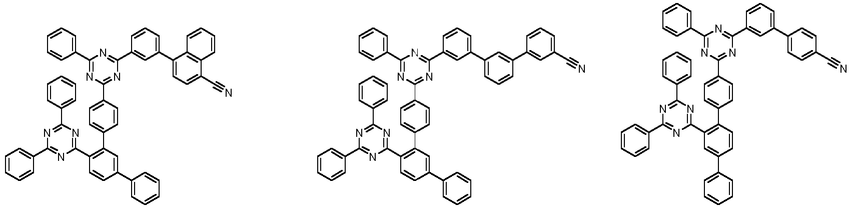

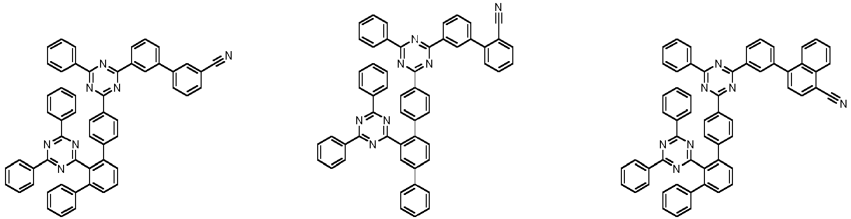

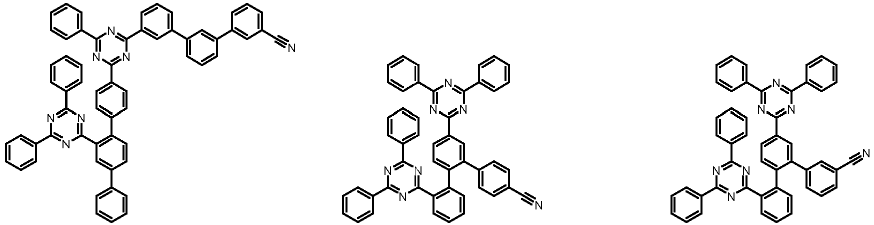

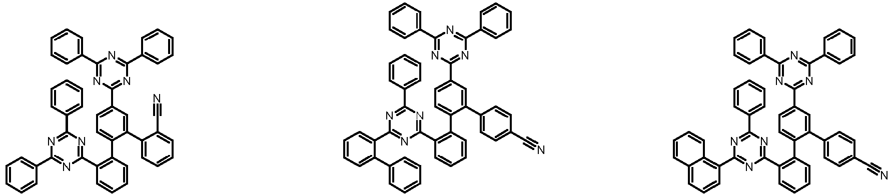

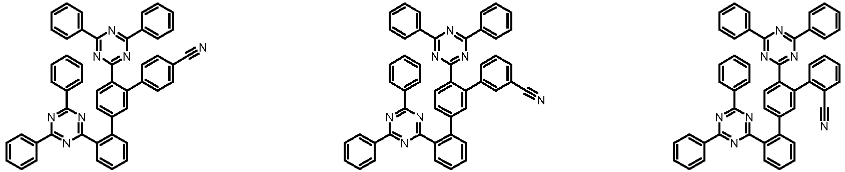

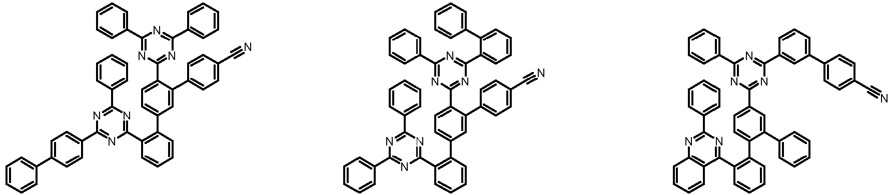

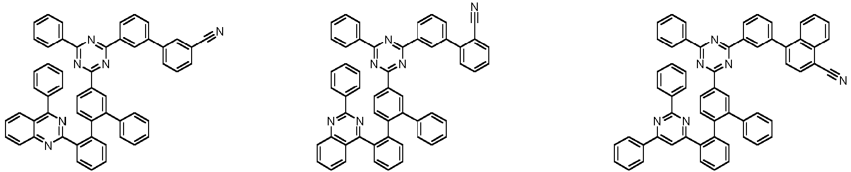

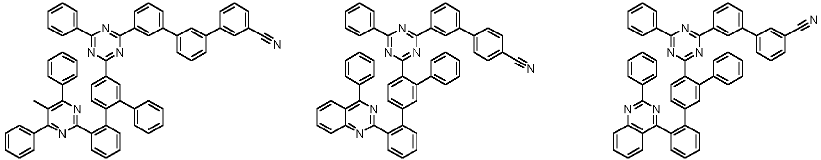

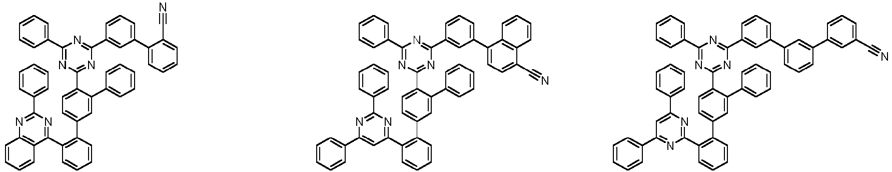

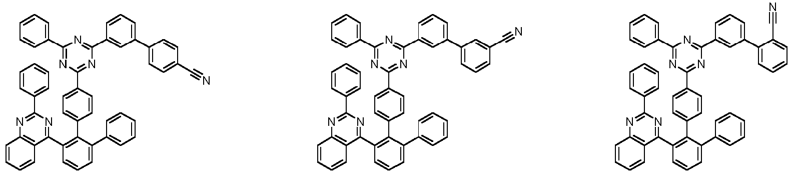

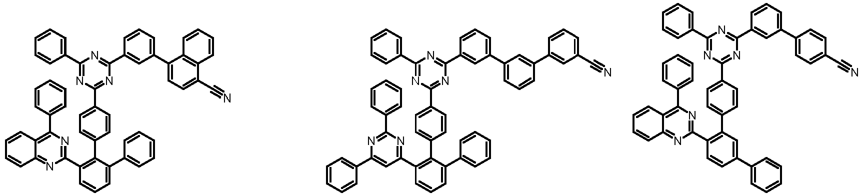

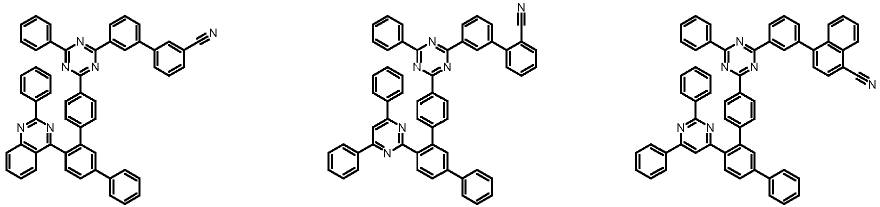

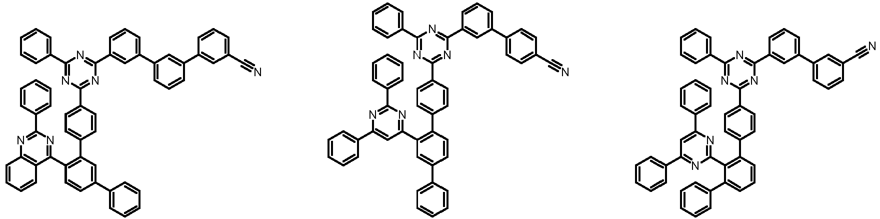

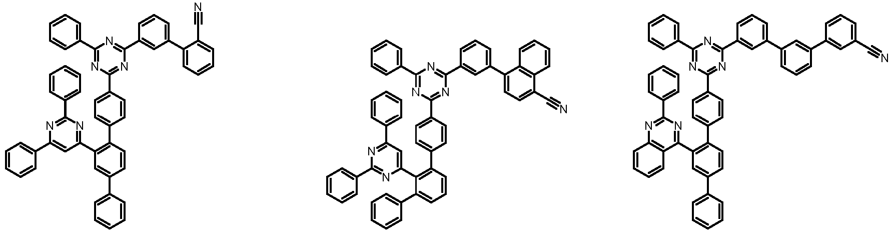

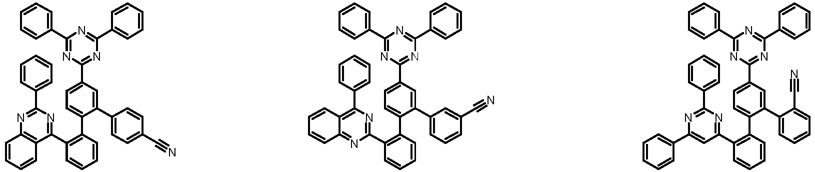

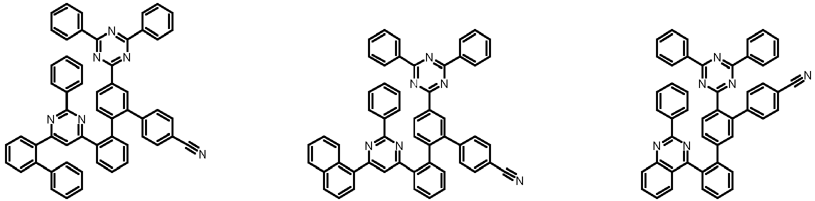

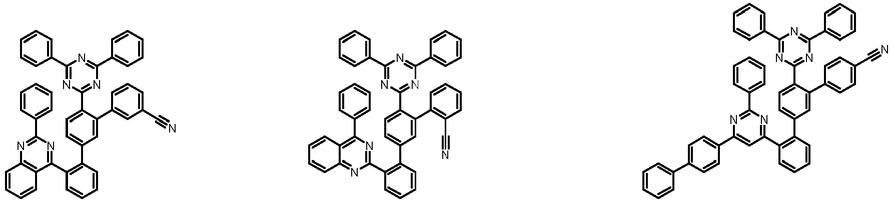

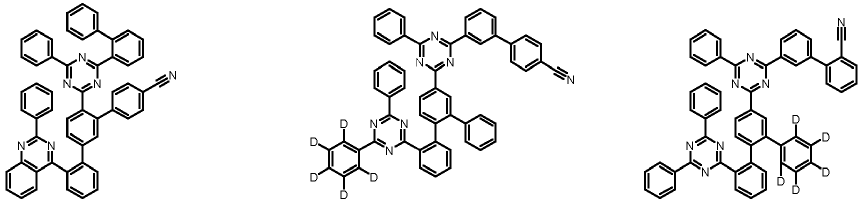

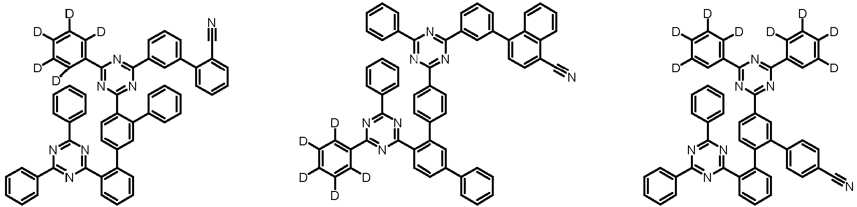

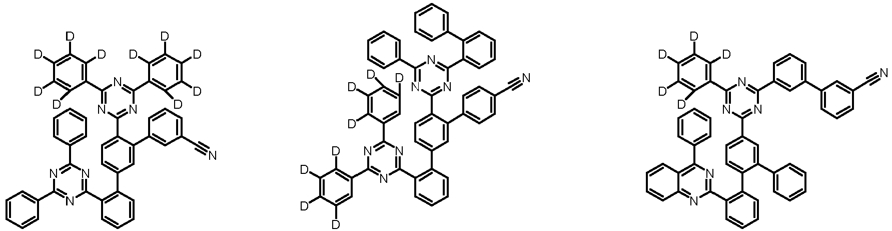

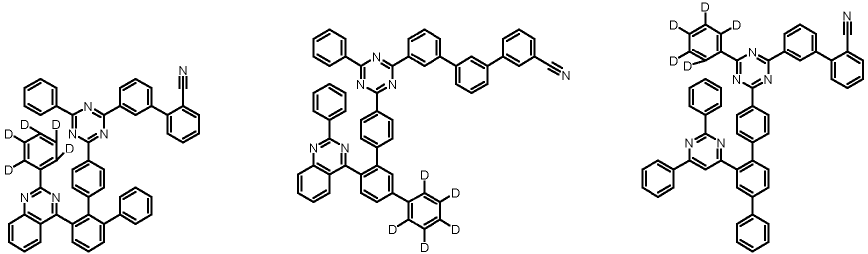

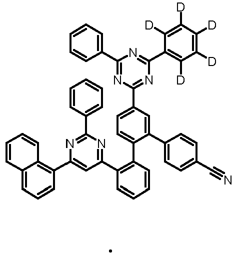

12. An organic light emitting device comprising: a first electrode; a second electrode that is provided opposite to the first electrode; and one or more organic material layers that are provided between the first electrode and the second electrode, wherein at least one layer of the organic material layers comprises the compound as defined in any one of claims 1 to 11.

13. An organic light emitting device of claim 12, wherein:
the organic material layer comprising the compound is an electron transport layer, an electron injection layer, or an electron transport and injection layer.

## Patentansprüche

1. Verbindung, dargestellt durch die folgende chemische Formel 1: 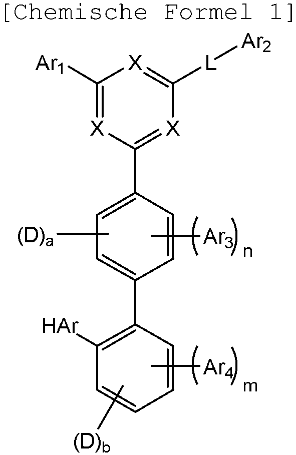
worin in der chemischen Formel 1
jedes X unabhängig N, CH oder CD ist, mit der Maßgabe, dass zwei oder mehr der X N sind,
L eine Einfachbindung oder substituiertes oder unsubstituiertes C₆₋₆₀-Arylen ist,
Ar₁ und Ar₂ jeweils unabhängig substituiertes oder unsubstituiertes C₆₋₆₀-Aryl sind,
Ar₃ und Ar₄ jeweils unabhängig Phenyl sind, das unsubstituiert oder mit mindestens einem Deuterium substituiert ist,
eines von Ar₁ bis Ar₄ mit Cyano substituiert ist,
HAr Pyrimidinyl ist, das unsubstituiert oder mit 1 bis 3 Substituentengruppen substituiert ist, die jeweils unabhängig ausgewählt sind aus der Gruppe bestehend aus Deuterium, substituiertem oder unsubstituiertem C₁₋₆₀-Alkyl und substituiertem oder unsubstituiertem C₆₋₆₀-Aryl; Triazinyl, das mit zwei substituierten oder unsubstituierten C₆₋₆₀-Arylen substituiert ist; oder Chinazolinyl, das mit einem substituierten oder unsubstituierten C₆₋₆₀-Aryl substituiert ist,
D Deuterium bedeutet,
a und b jeweils unabhängig eine ganze Zahl von 0 bis 4 sind,
n und m jeweils unabhängig 0 oder 1 sind,
mit der Maßgabe, dass n+m 1 ist,
a+n eine ganze Zahl von 0 bis 4 ist und
b+m eine ganze Zahl von 0 bis 4 ist.

2. Verbindung gemäß Anspruch 1, worin
die chemische Formel 1 dargestellt ist durch eine der folgenden chemischen Formeln 1-1 bis 1-3: 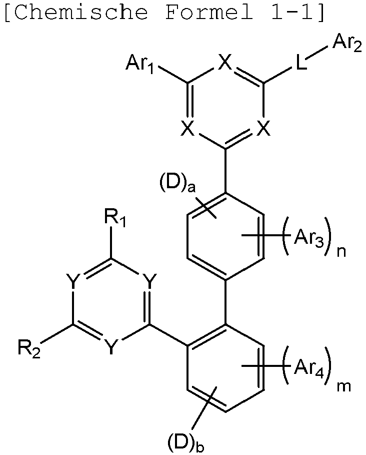 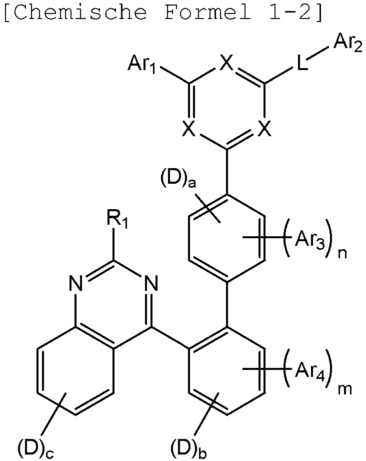 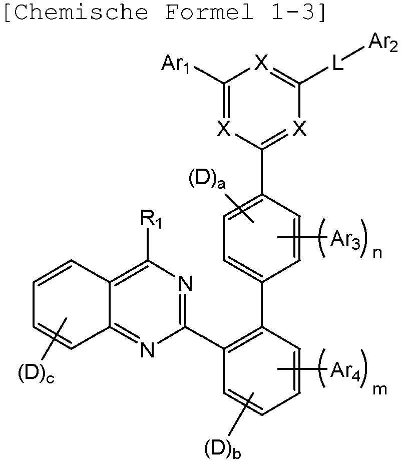
worin in den chemischen Formeln 1-1 bis 1-3
X, L, Ar₁, Ar₂, Ar₃, Ar₄, D, a, b, n und m wie in Anspruch 1 definiert sind,
Y N oder CR' ist, mit der Maßgabe, dass zwei oder mehr der Y N sind,
R' Wasserstoff, Deuterium, substituiertes oder unsubstituiertes C₁₋₆₀ Alkyl oder substituiertes oder unsubstituiertes C₆₋₆₀ Aryl ist,
R₁ und R₂ jeweils unabhängig substituiertes oder unsubstituiertes C₆₋₆₀ Aryl sind, und
c eine ganze Zahl von 0 bis 4 ist.

3. Verbindung gemäß Anspruch 1, worin
die chemische Formel 1 dargestellt ist durch eine der folgenden chemischen Formeln 1-4 bis 1-9: 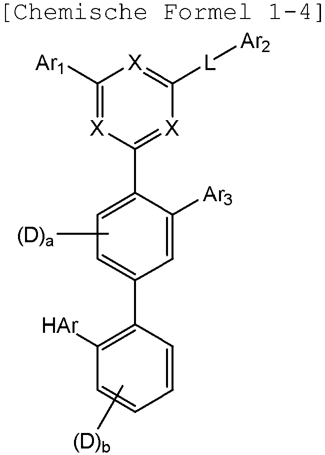 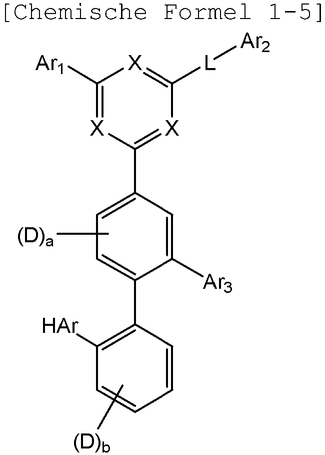 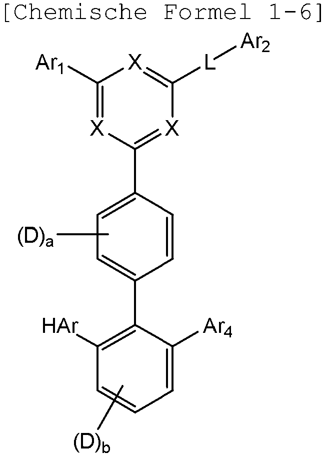 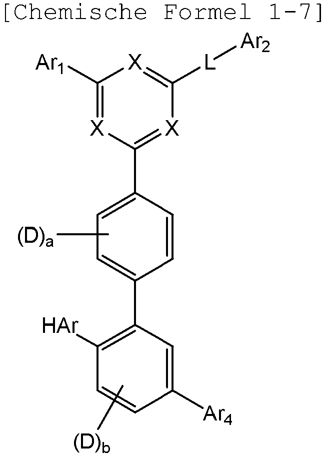 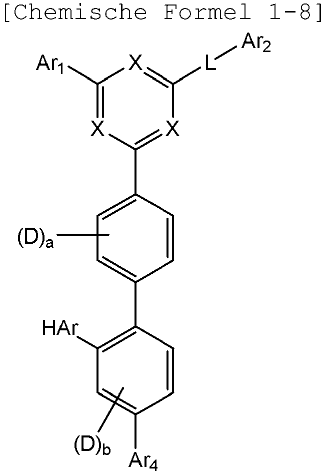 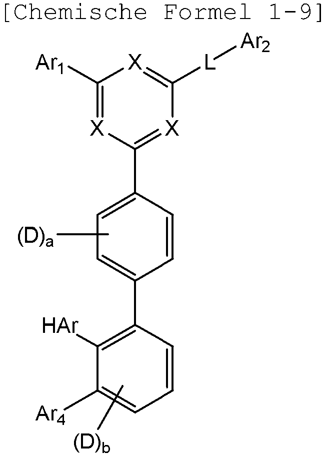
worin in den chemischen Formeln 1-4 bis 1-9
X, L, Ar₁, Ar₂, Ar₃, Ar₄, HAr, D, a und b wie in Anspruch 1 definiert sind.

4. Verbindung gemäß Anspruch 1, worin
L eine Einfachbindung, Phenylen oder Biphenyldiyl ist, worin Phenylen und Biphenyldiyl unsubstituiert oder mit mindestens einem Deuterium substituiert sind.

5. Verbindung gemäß Anspruch 1, worin
Ar₁ Phenyl ist, das unsubstituiert oder mit mindestens einem Deuterium substituiert ist.

6. Verbindung gemäß Anspruch 1, worin
Ar₂ Phenyl, Biphenylyl oder Naphthyl ist,
worin Ar₂ unsubstituiert oder mit mindestens einem Deuterium substituiert ist.

7. Verbindung gemäß Anspruch 1, worin
L Phenylen oder Biphenyldiyl ist,
worin L unsubstituiert oder mit mindestens einem Deuterium substituiert ist, und
Ar₂ Phenyl ist, das mit Cyano substituiert ist, oder mit Cyano substituiertes Naphthyl ist,
worin Ar₂ mit Deuterium substituiert oder unsubstituiert ist.

8. Verbindung gemäß Anspruch 1, worin
L eine Einfachbindung ist,
Ar₂ Phenyl oder Biphenylyl ist,
worin Ar₂ unsubstituiert oder mit mindestens einem Deuterium substituiert ist, und
Ar₃ Phenyl ist, das mit Cyano substituiert ist,
worin Ar₃ mit Deuterium substituiert oder unsubstituiert ist.

9. Verbindung gemäß Anspruch 2, worin
R' Wasserstoff, Deuterium oder Methyl ist, das unsubstituiert oder mit mindestens einem Deuterium substituiert ist.

10. Verbindung gemäß Anspruch 2, worin
R₁ und R₂ jeweils unabhängig Phenyl, Biphenylyl oder Naphthyl sind,
worin R₁ und R₂ unsubstituiert oder mit mindestens einem Deuterium substituiert sind.

11. Verbindung gemäß Anspruch 1, worin
die durch die chemische Formel 1 dargestellte Verbindung eine Verbindung ist, die ausgewählt ist aus der Gruppe bestehend aus Verbindungen der folgenden Strukturformeln:

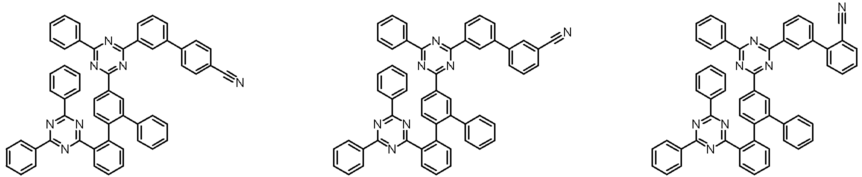

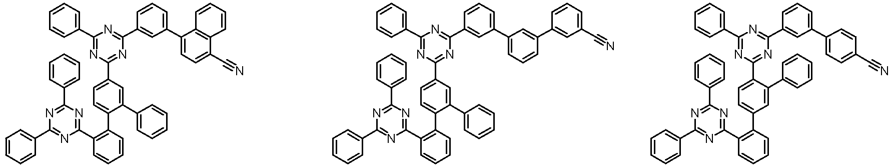

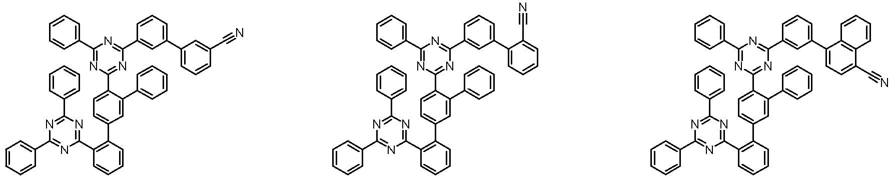

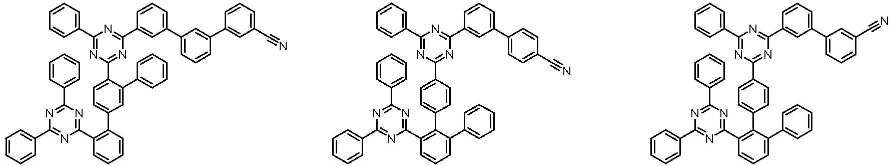

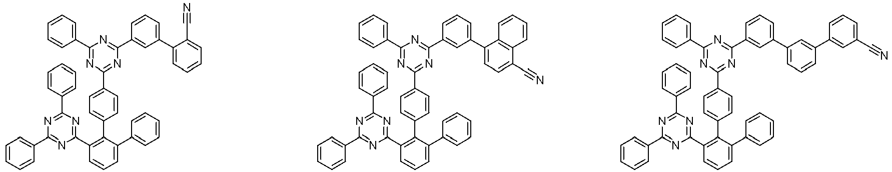

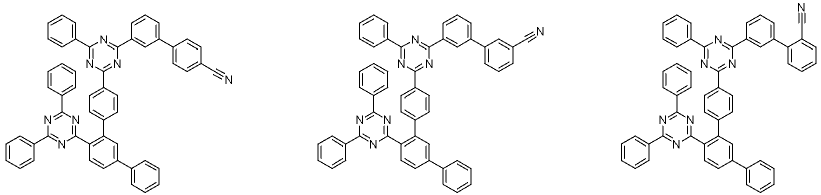

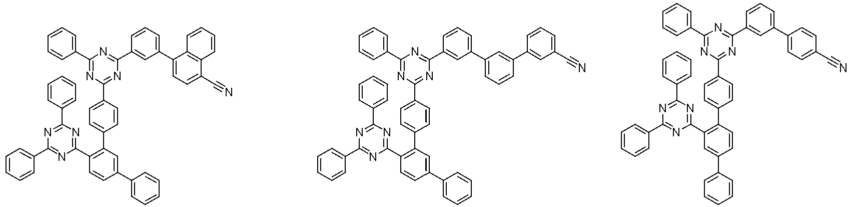

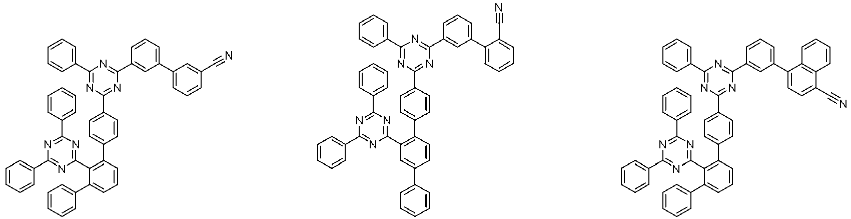

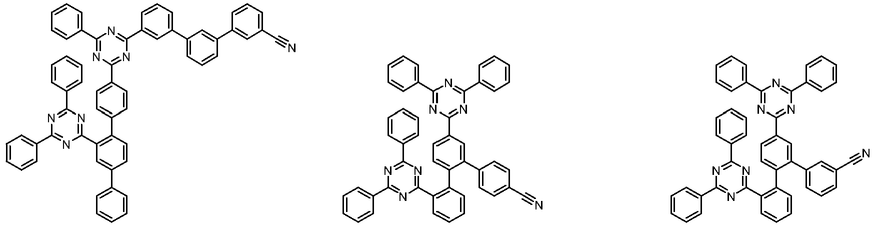

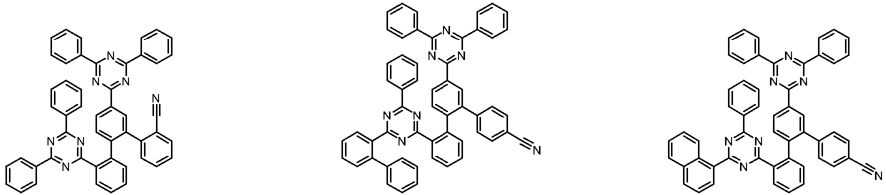

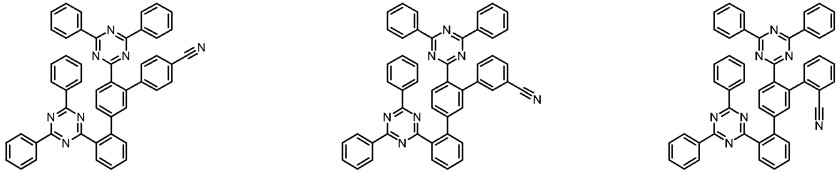

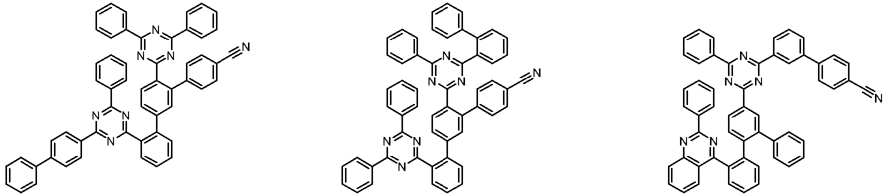

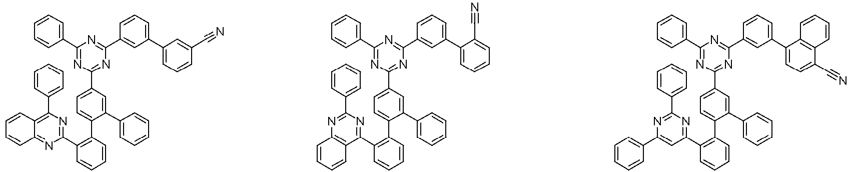

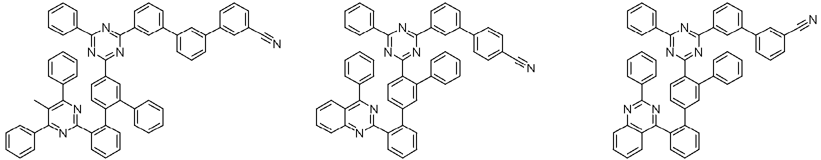

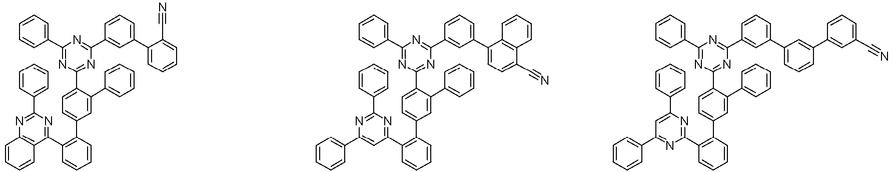

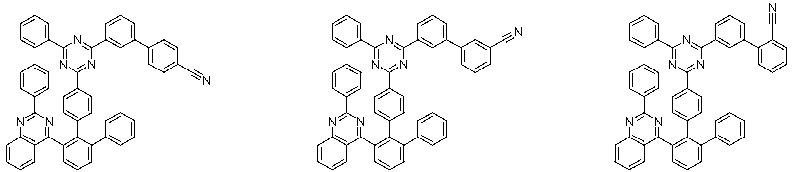

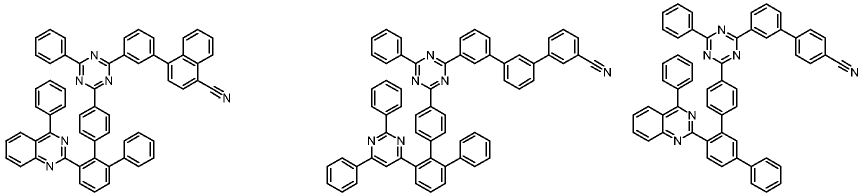

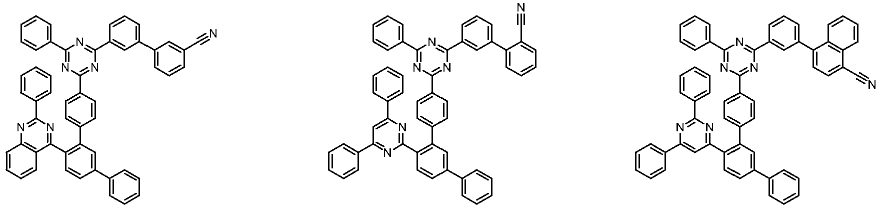

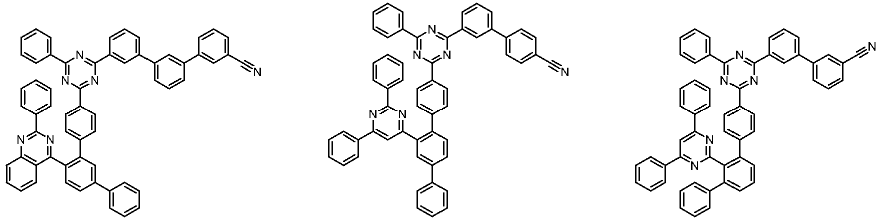

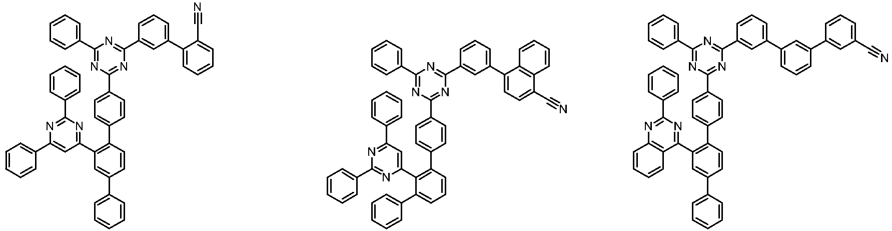

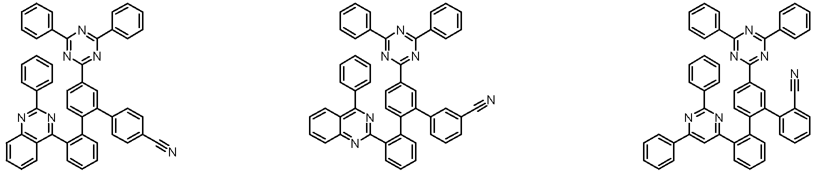

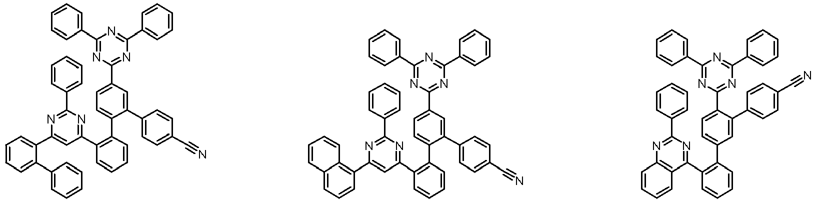

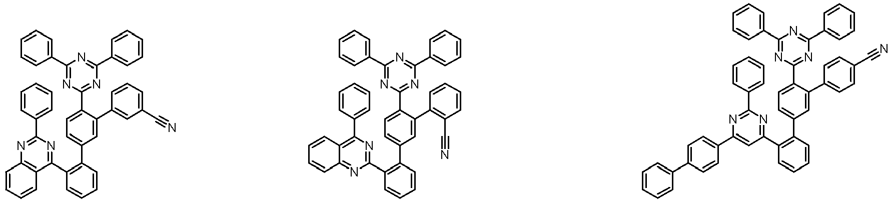

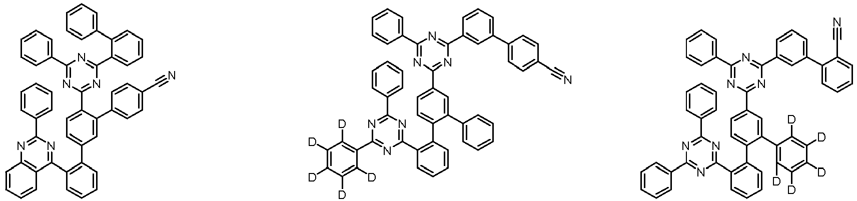

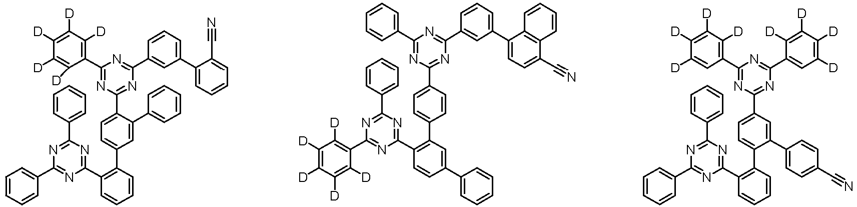

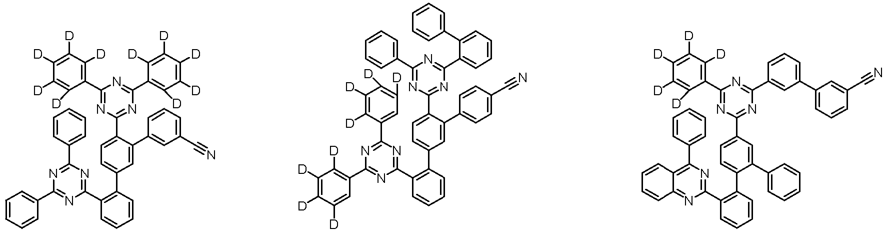

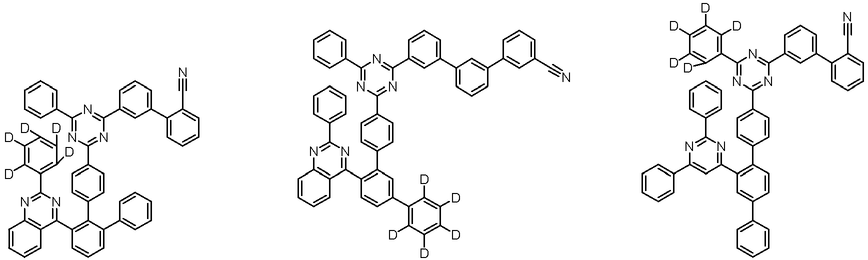

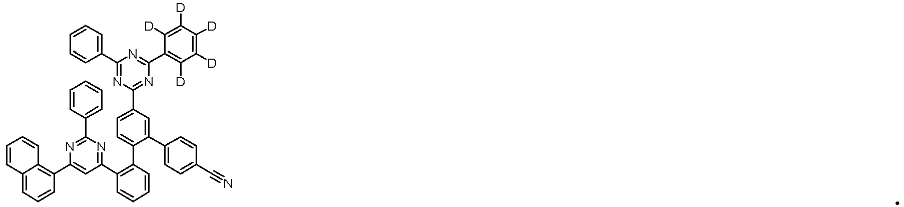

12. Organische Licht emittierende Vorrichtung, umfassend eine erste Elektrode; eine zweite Elektrode, die gegenüber der ersten Elektrode angeordnet ist; und eine oder mehrere Schichten aus organischem Material, die zwischen der ersten Elektrode und der zweiten Elektrode angeordnet sind, wobei mindestens eine Schicht der Schichten aus organischem Material die Verbindung, wie in mindestens einem der Ansprüche 1 bis 11 definiert, umfasst.

13. Organische Leuchtvorrichtung gemäß Anspruch 12, worin die Schicht aus organischem Material, die die Verbindung umfasst, eine Elektronentransportschicht, eine Elektroneninjektionsschicht oder eine Elektronentransport- und -injektionsschicht ist.

## Revendications

1. Composé représenté par la formule chimique 1 suivante : 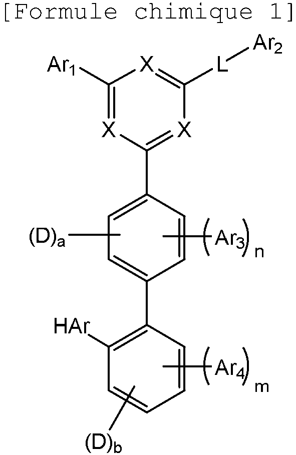
dans laquelle, dans la formule chimique 1,
chaque X représente indépendamment N, CH ou CD, à condition que deux ou plusieurs des X représentent N, L est une liaison simple ou un groupe arylène en C6-60 substitué ou non substitué,
Ar₁ et Ar₂ sont chacun indépendamment un groupe aryle en C6-60 substitué ou non substitué,
Ar₃ et Ar₄ sont chacun indépendamment un groupe phényle non substitué ou substitué par au moins un deutérium, l'un quelconque des groupes Ar₁ à Ar₄ est substitué par un groupe cyano,
HAr est un groupe pyrimidinyle non substitué ou substitué par 1 à 3 groupes substituants choisis chacun indépendamment dans le groupe constitué par le deutérium, un groupe alkyle en C1-60 substitué ou non substitué et un groupe aryle en C6-60 substitué ou non substitué ; un groupe triazinyle qui est substitué par deux groupes aryle en C6-60 substitués ou non substitués ; ou un groupe quinazolinyle qui est substitué par un groupe aryle en C6-60 substitué ou non substitué,
D représente le deutérium,
a et b représentent chacun indépendamment un nombre entier de 0 à 4,
n et m représentent chacun indépendamment 0 ou 1,
à condition que n+m soit égal à 1,
a+n soit un nombre entier de 0 à 4, et
b+m soit un nombre entier de 0 à 4.

2. Le composé de la revendication 1, dans lequel :
la formule chimique 1 est représentée par l'une quelconque des formules chimiques 1-1 à 1-3 suivantes : 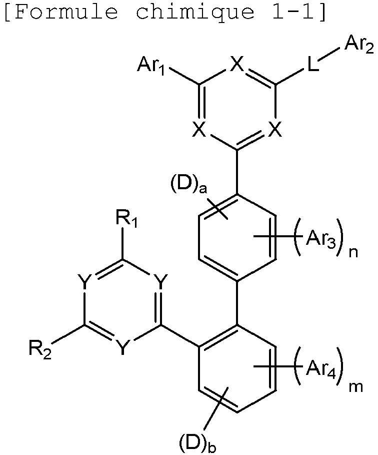 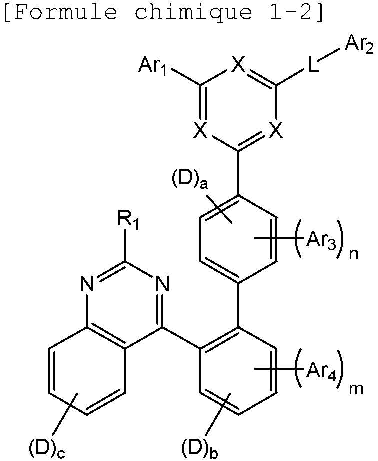 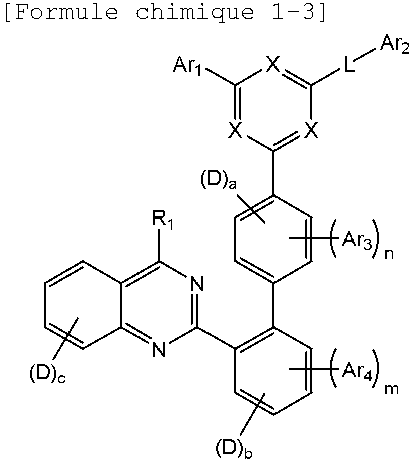
dans lesquelles, dans les formules chimiques 1-1 à 1-3, X, L, Ar₁, Ar₂, Ar₃, Ar₄, D, a, b, n et m sont tels que définis dans la revendication 1,
Y est N ou CR', à condition que deux ou plusieurs des Y soient N,
R' est un atome d'hydrogène, un atome de deutérium, un groupe alkyle en C1-60 substitué ou non substitué, ou un groupe aryle en C6-60 substitué ou non substitué,
R₁ et R₂ sont chacun indépendamment un groupe aryle en C6-60 substitué ou non substitué, et
c est un nombre entier compris entre 0 et 4.

3. Le composé de la revendication 1, dans lequel :
la formule chimique 1 est représentée par l'une quelconque des formules chimiques 1-4 à 1-9 suivantes : 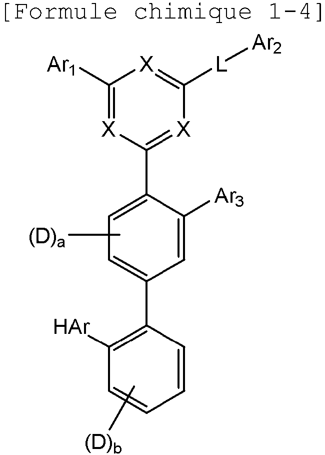 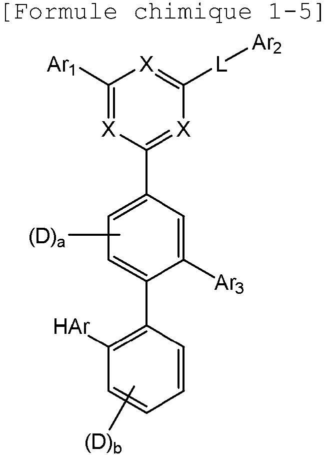 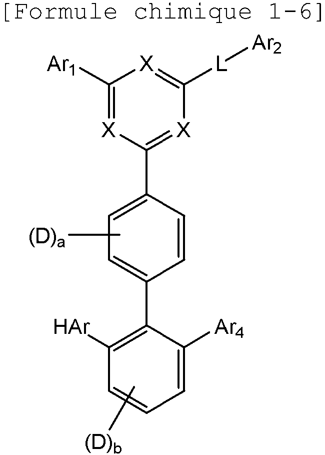 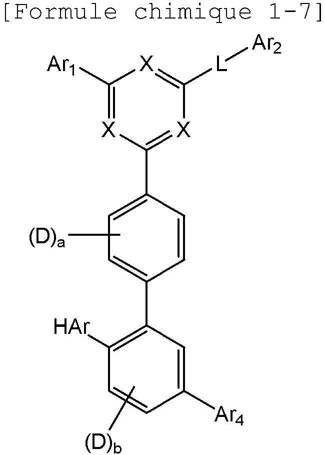 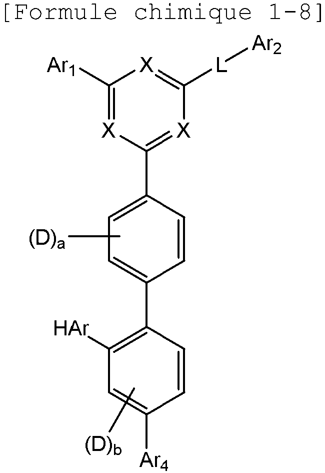 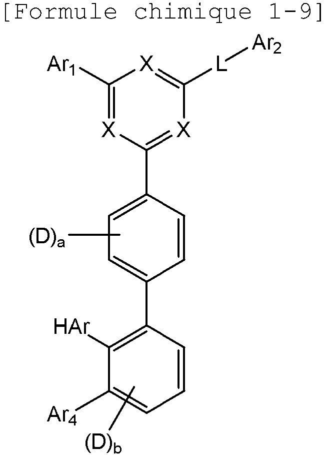
dans lesquelles, dans les formules chimiques 1-4 à 1-9, X, L, Ar₁, Ar₂, Ar₃, Ar₄, HAr, D, a et b sont tels que définis dans la revendication 1.

4. Le composé de la revendication 1, dans lequel :
L est une liaison simple, un phénylène ou un biphényldiyl,
dans lequel le phénylène et le biphényldiyl sont non substitués ou substitués par au moins un deutérium.

5. Le composé de la revendication 1, dans lequel :
Ar₁ est un phényle non substitué ou substitué par au moins un deutérium.

6. Le composé de la revendication 1, dans lequel :
Ar₂ est un groupe phényle, biphénylyle ou naphtyle, dans lequel Ar₂ est non substitué ou substitué par au moins un deutérium.

7. Le composé de la revendication 1, dans lequel :
L est un groupe phénylène ou biphényldiyl,
dans lequel L est non substitué ou substitué par au moins un deutérium, et
Ar₂ est un groupe phényle substitué par un groupe cyano, ou un groupe naphtyle substitué par un groupe cyano, dans lequel Ar₂ est substitué ou non substitué par un atome de deutérium.

8. Le composé selon la revendication 1, dans lequel :
L est une liaison simple,
Ar₂ est un groupe phényle ou biphénylyle,
dans lequel Ar₂ est non substitué ou substitué par au moins un atome de deutérium, et
Ar₃ est un groupe phényle substitué par un groupe cyano, dans lequel Ar₃ est substitué ou non substitué par un atome de deutérium.

9. Le composé selon la revendication 2, dans lequel :
R' est un atome d'hydrogène, un atome de deutérium ou un groupe méthyle non substitué ou substitué par au moins un atome de deutérium.

10. Le composé de la revendication 2, dans lequel :
R₁ et R₂ sont chacun indépendamment un groupe phényle, biphénylyle ou naphtyle,
dans lequel les groupes R₁ et R₂ sont non substitués ou substitués par au moins un deutérium.

11. Le composé de la revendication 1, dans lequel :
le composé représenté par la formule chimique 1 est choisi parmi le groupe constitué des composés ayant les formules structurales suivantes :

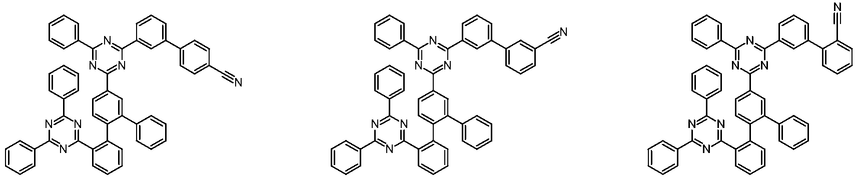

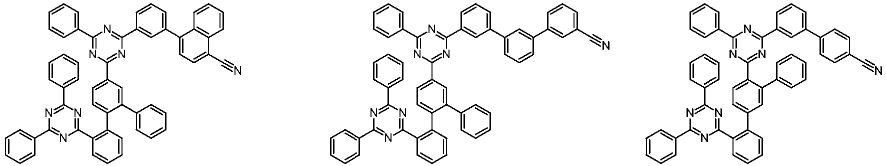

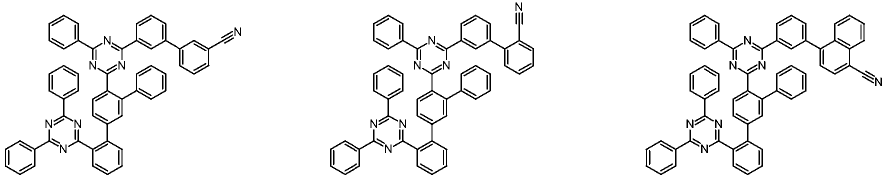

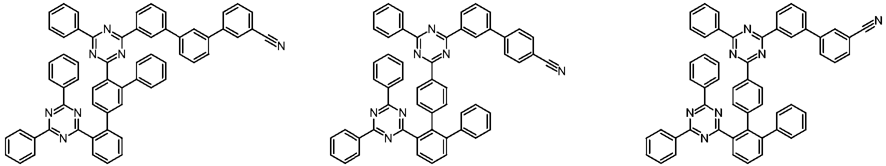

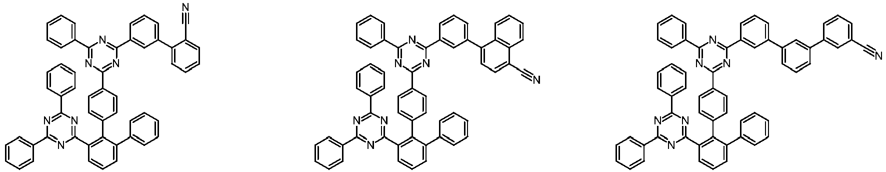

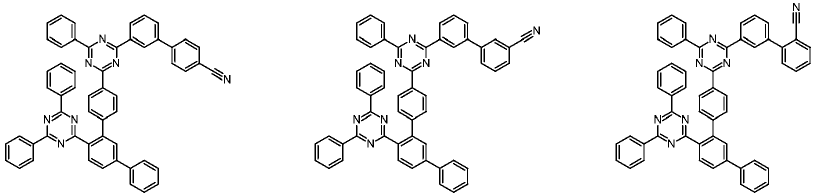

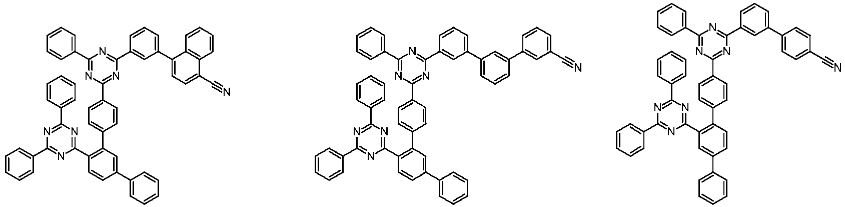

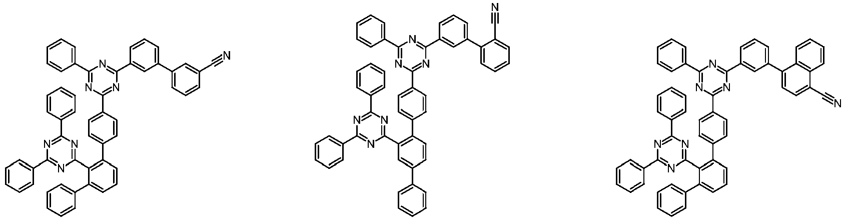

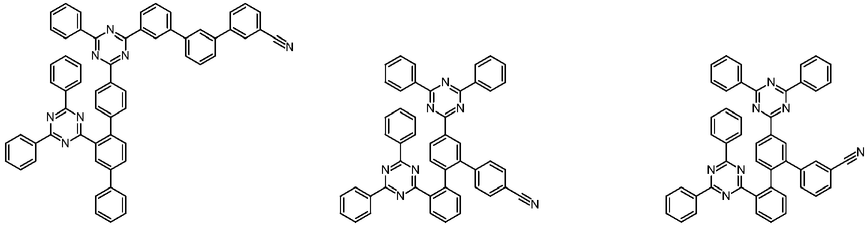

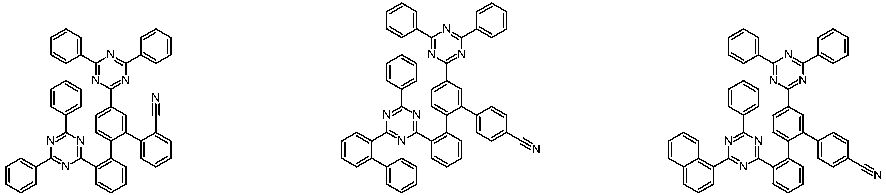

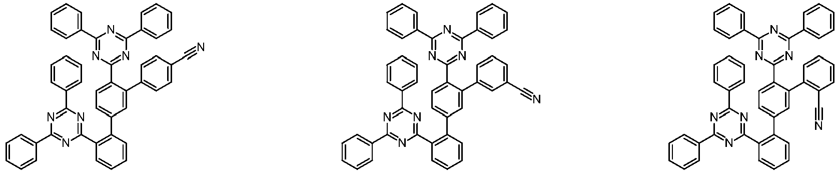

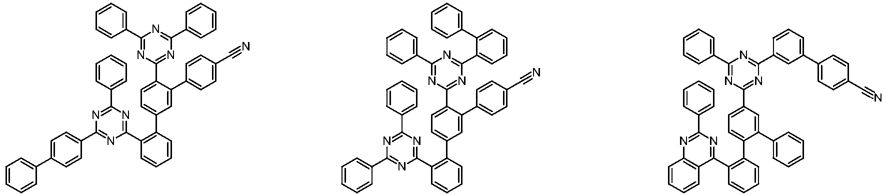

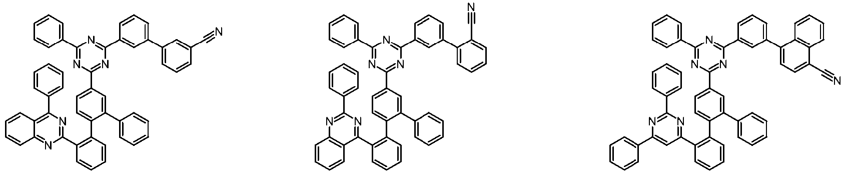

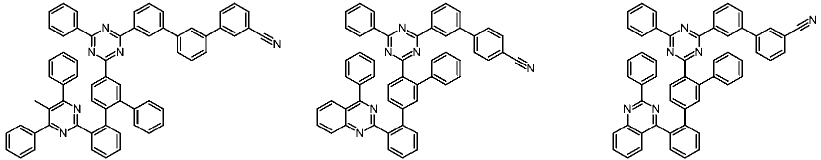

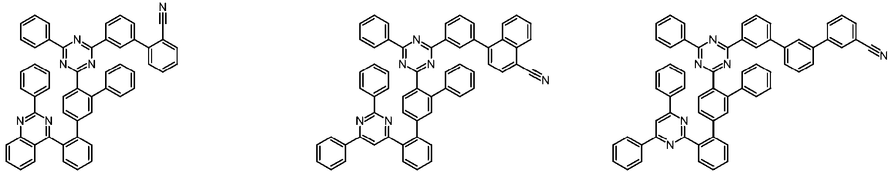

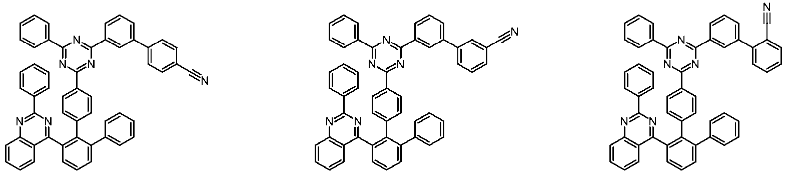

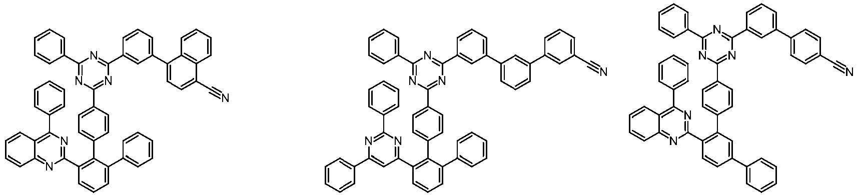

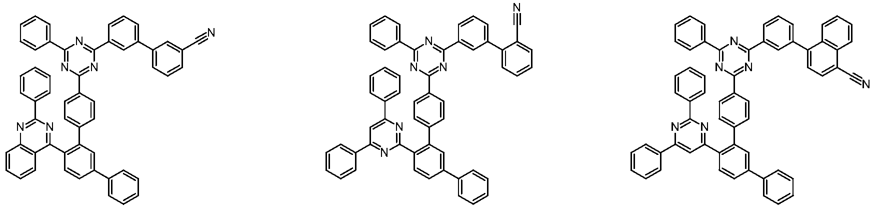

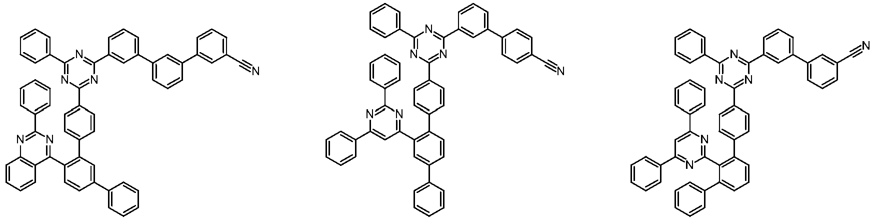

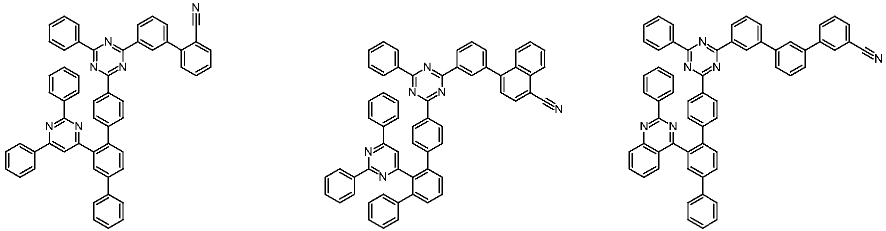

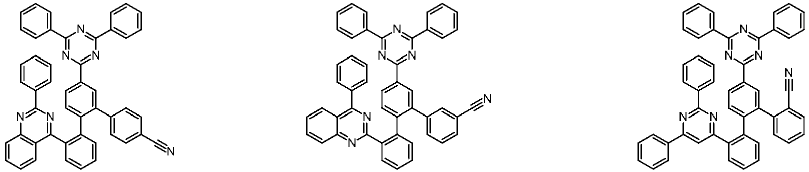

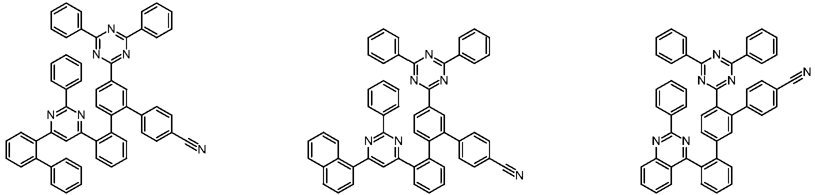

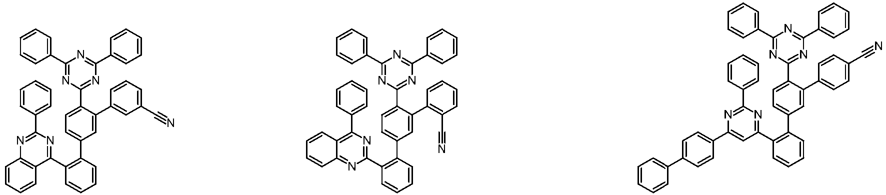

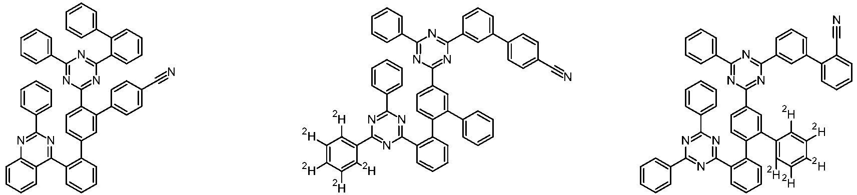

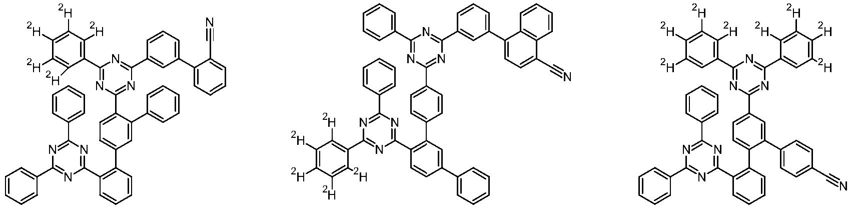

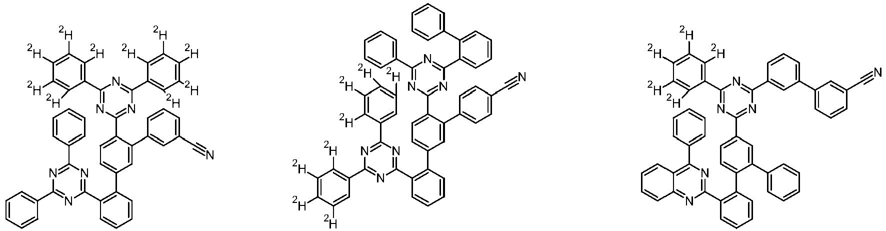

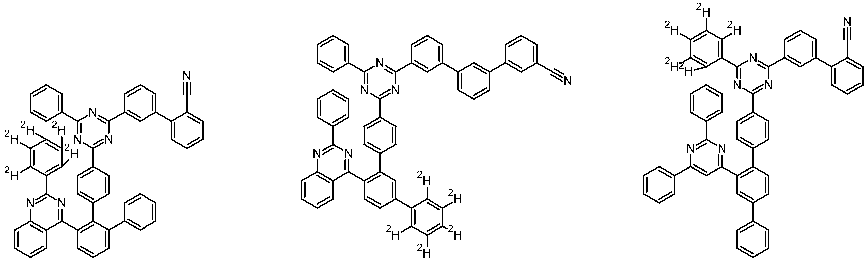

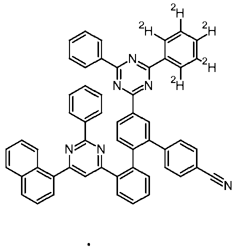

12. Dispositif électroluminescent organique comprenant : une première électrode ; une deuxième électrode disposée à l'opposé de la première électrode ; et une ou plusieurs couches de matériau organique disposées entre la première électrode et la deuxième électrode, dans lequel au moins une couche parmi les couches de matériau organique comprend le composé tel que défini dans l'une quelconque des revendications 1 à 11.

13. Dispositif électroluminescent organique selon la revendication 12, dans lequel :
la couche de matériau organique comprenant le composé est une couche de transport d'électrons, une couche d'injection d'électrons ou une couche de transport et d'injection d'électrons.
